# EUROPEAN PATENT SPECIFICATION

(11) **EP 4 419 039 B1**
(45) Date of publication and mention of the grant of the patent: **11.03.2026**
(21) Application number: 22800354.7
(22) Date of filing: 18.10.2022
(51) Int. Cl.: A61B 90/00, A61B 17/16, A61B 50/00, A61B 50/30

(54) **ATTACHMENT AND SYSTEM FOR TRACKING A SURGICAL INSTRUMENT**
AUFSATZ UND SYSTEM ZUR VERFOLGUNG EINES CHIRURGISCHEN INSTRUMENTS
ACCESSOIRE ET SYSTÈME DE SUIVI D'UN INSTRUMENT CHIRURGICAL

(30) Priority: 18.10.2021 US 202163257066 P; 10.03.2022 US 202263318436 P; 04.10.2022 US 202263413014 P
(43) Date of publication of application: 28.08.2024
(73) Proprietor: Stryker European Operations Limited, Carrigtwohill, Co. Cork T45 HX08 (IE)
(72) Inventor: CUSHEN, Patrick Eoin, Cork Shanagarry, P25AX88 (IE); GOODENOUGH, Dave, Marcellus, MI 49067 (US); GOSSENS, Caleb, Ravenna, MI 49451 (US); HOODA, Ritesh, Rohtak, Haryana (IN); LAMBARTH, Clifford Edwin, Portage, MI 49002 (US); MISHRA, Hemant, Faridabad, Haryana 121004 (IN); SNODGRASS, John A., Plainwell, MI 49080 (US); WALEN, James G., Portage, MI 49024 (US); FRANCK, Brian, Kalamazoo, MI 49009 (US); MAILE, David Alexander, Kalamazoo, MI 49009 (US)
(74) Representative: Röthinger, Rainer
(86) International application number: PCT/IB2022/060009
(87) International publication number: WO 2023/067503

(56) References cited:
- EP-A1- 1 570 791
- EP-A1- 2 574 301
- EP-A2- 1 444 962
- US-A1- 2011 263 971
- US-A1- 2015 031 985
- US-A1- 2019 150 901
- US-A1- 2020 337 785
- US-B1- 6 434 507

## Description

### BACKGROUND

Certain accessories, such as a tracking marker in one non-limiting example, have been permanently fixed to, and may comprise part of, an associated surgical instrument. There is a need for an accessory mount for use with a surgical instrument that allows easy installation and removal of such an accessory and provides consistent alignment of the accessory with the surgical instrument. EP 1 570 791 A1 discusses a surgical instrument for use in a computer assisted navigation system is provided. US 6 434 507 B1 discusses an apparatus for use with a surgical navigation system.

### SUMMARY

A tracker attachment is defined by independent claim 1, to which the reader should now refer. Claim 9 defines an associated system. The dependent claims define specific embodiments.

In one example an attachment for a surgical instrument is disclosed. The surgical instrument may include a surgical handpiece. The attachment may include an attachment body extending from a proximal end to a distal end. The proximal end may be configured for engaging the surgical handpiece. The attachment body may comprise a bushing portion arranged at the proximal end of the attachment body and engageable with a distal end of the surgical handpiece. The bushing portion has a first diameter. The attachment body may further comprise a mount flange arranged distally of the bushing portion. The mount flange has a second diameter greater than the first diameter of the bushing portion. The attachment may further include a tool chuck coupled to the attachment body and configured to receive a material removal tool arranged at a working end of the surgical instrument. The attachment may further include a driveshaft supported in the attachment body and operably coupled to the tool chuck. The driveshaft may be rotatable about an instrument axis for transferring power from the surgical handpiece to the material removal tool. The attachment may further include a cantilever arm coupled to the attachment body adjacent to the mount flange. The cantilever arm extends in a proximal direction to an accessory mount. The accessory mount may be positioned proximally of the bushing portion.

In a second example a system for tracking the position of a rotary cutting tool is disclosed. The system may include a rotary cutting tool, a surgical handpiece including a motor, and a surgical instrument attachment. The surgical instrument attachment may comprise an attachment body extending from a proximal end to a distal end. The proximal end may be configured for engaging the surgical handpiece. The surgical instrument attachment may further include a cantilever arm coupled to the attachment body and extending in a proximal direction to an accessory mount. The accessory mount may be arranged proximally of the proximal end of the attachment body and spaced from the surgical handpiece. The system may further include a tracker. The tracker may include a tracker frame and at least three optical markers coupled to the tracker frame. The tracker may further include an accessory socket coupled to the tracker frame. The accessory socket may be configured to engage the accessory mount. The tracker may be arranged proximally of the attachment body when the accessory socket is coupled to the accessory mount.

In a third example a coupling arrangement for coupling a surgical instrument tracker to a surgical instrument is disclosed. The coupling arrangement may include an accessory mount and an accessory socket. The accessory mount may be coupled to the surgical instrument and the accessory socket may be coupled to the surgical instrument tracker. The accessory mount may include a trunk portion having a top surface and defining a clamping hole in the top surface. The accessory mount may further include a first lateral wing portion and a second lateral wing portion protruding from opposing sides of the trunk portion transverse to the top surface. The first lateral wing portion may have a first lateral face and the second lateral wing portion may have a second lateral face. The first lateral face and the second lateral face may be symmetrically angled about a longitudinal plane of the surgical instrument. The accessory mount may further include a plurality of alignment pegs protruding from the top surface. The accessory socket may include a clamp surface defining a fastener hole and a plurality of alignment channels recessed in the clamp surface and configured to complementarily engage the plurality of alignment pegs. The accessory socket may further include a first socket wall depending from the clamp surface and having a first rail surface spaced from the clamp surface and a first ramp surface extending between the first rail surface and the clamp surface. The accessory socket may further include a second socket wall depending from the clamp surface and having a second rail surface spaced from the clamp surface and a second ramp surface extending between the second rail surface and the clamp surface. The system may further include a clamp fastener disposed in the fastener hole and engageable with the clamping hole. The clamp fastener may be configured to bias the top surface of the trunk portion toward the clamp surface of the accessory socket such that the alignment pegs engage the alignment channels.

In a fourth example a sterile packaging system for a tracker for surgical navigation is disclosed. The sterile packaging system may include a sterile tracker and a blister pack. The sterile tracker may include a tracker frame defining a battery receptacle. The sterile tracker may further include an electrical circuit comprising a positive terminal and a negative terminal arranged in the battery receptacle and a plurality of tracking markers in electrical communication with the positive terminal and the negative terminal. The sterile tracker may further include a battery disposed in the battery receptacle between the positive terminal and the negative terminal and an isolating member disposed between the battery and each of the positive terminal the negative terminal. The blister pack may include a shell portion defining a sterile interior shaped receive the sterile tracker. The blister pack may further include a sealing film in cooperation with the shell portion and disposed over the sterile interior to seal the sterile tracker within the blister pack.

Any of the above examples can be combined in full or in part. Any features of the above examples can be combined in full or in part. Any of the above implementations for any example can be combined with any other aspect. Any of the above implementations can be combined with any other implementation whether for the same aspect or a different example. This summary introduces a selection of concepts in a simplified form that are further described below in the Detailed Description below. This summary is not intended to limit the scope of the claimed subject matter nor identify key features or essential features of the claimed subject matter.

### BRIEF DESCRIPTION OF THE DRAWINGS

Advantages of the present disclosure will be readily appreciated as the same becomes better understood by reference to the following detailed description when considered in connection with the accompanying drawings.
FIG. 1 is an exemplary surgical suite showing a surgical navigation system.
FIG. 2 is a perspective view of a user holding a surgical instrument having an attachment and a tracker coupled thereto.
FIG. 3 is another perspective view of the surgical instrument and tracker of FIG. 2.
FIG. 4 is a perspective view of the surgical instrument with the tracker in a removed position and showing a tracker in a partially removed in phantom.
FIG. 5 is a perspective view of the tracker and a coupling arrangement having an accessory mount and an accessory socket.
FIG. 6 is a bottom-side perspective view of the coupling arrangement and the tracker of FIG 5.
FIG. 7 is a cross-sectional view of the coupling arrangement.
FIG. 8 is a cross-sectional view of the tracker, the coupling arrangement, and the attachment.
FIG. 9 is a close-up cross-sectional view of the tracker showing the accessory socket of the coupling arrangement.
FIG. 10 is a perspective cross-sectional view of the tracker and the coupling arrangement.
FIG. 11 is a cross-sectional perspective view of the tracker and an isolating strip.
FIG. 12 is a cross-section view of the tracker and the accessory socket.
FIG. 13 is a bottom-side perspective view of the tracker and the accessory socket.
FIG. 14 is a perspective view of the attachment for the surgical instrument and the accessory mount.
FIG. 15 is a proximal-end view of the attachment and the accessory mount.
FIG. 16 is another perspective view of the accessory mount.

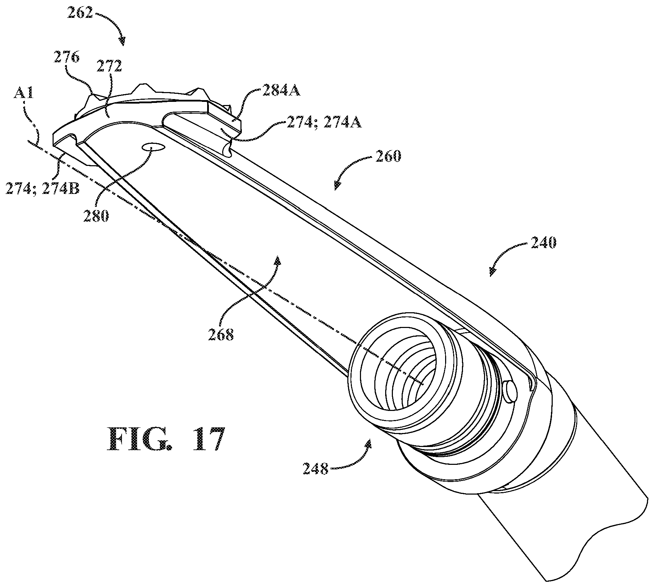

FIG. 17 another perspective view of the accessory mount.
FIG. 18 is a perspective view of a surgical instrument having an attachment and a tracker coupled thereto.
FIG. 19 is a close-up side view of the surgical instrument and tracker of FIG. 18 shown with the tracker partially removed from the surgical instrument.
FIG. 20 is a bottom-side perspective view of the tracker and a coupling arrangement having an accessory mount and an accessory socket.
FIG. 21 is another perspective view of the tracker and the coupling arrangement of FIG. 20.
FIG. 22 is a close-up cross-sectional view of the tracker showing the accessory mount of the coupling arrangement.
FIG. 23 is a perspective cross-sectional view of the tracker and the coupling arrangement.
FIG. 24 is another cross-sectional view of the tracker and the coupling arrangement.
FIG. 25 is a bottom-side perspective view of the tracker and accessory socket.
FIG. 26 is a bottom-side view of the tracker and accessory socket.
FIG. 27 is a bottom-side perspective view of the tracker and the accessory socket.
FIG. 28 is a partially cutaway perspective view of the tracker of FIGS. 2-12 disposed and supported inside a sterile packaging system and sterilized.
FIG. 29 is another partially cutaway perspective view of the sterile packaging system of FIG. 28 showing a shell portion and an insert portion.
FIG. 30 is an environmental view of the sterile packaging system showing a user removing the sterilized tracker and insert portion from the shell portion.
FIG. 31 is an environmental view of the sterile packaging system showing a user assembling the sterilized tracker to a surgical instrument without direct contact with the sterilized tracker.

### DETAILED DESCRIPTION

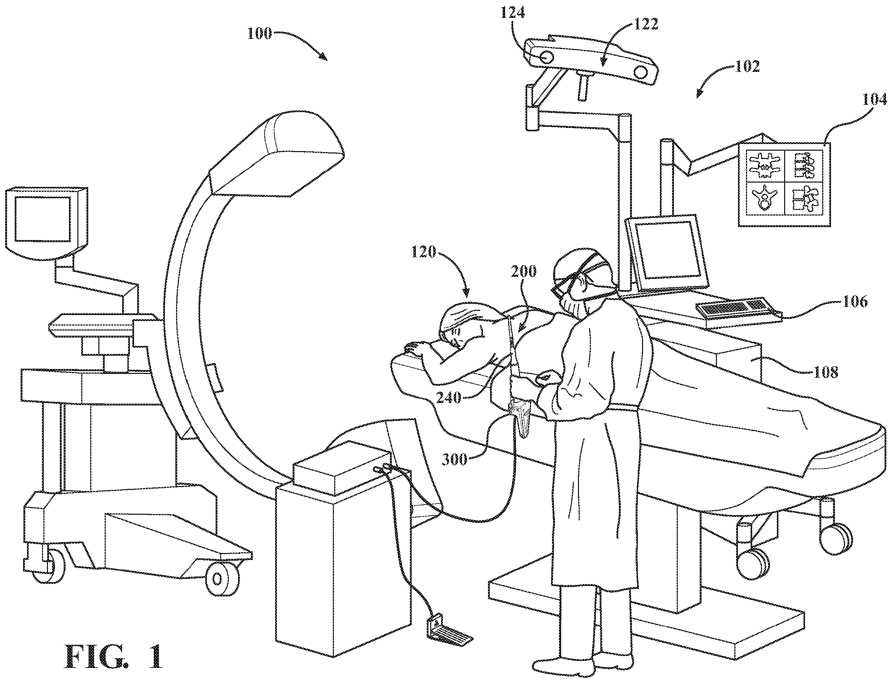

With reference to the figures, wherein like numerals indicate like parts throughout the several views, the present disclosure includes two exemplary implementations of a navigation tracker 300, 1300 (discussed in further detail below) and an instrument attachment 240, for a handheld surgical instrument 200 usable with a surgical navigation system 102 and a method for operating the tracker 300. FIG. 1 illustrates an exemplary surgical system 100 that may comprise the surgical navigation system 102 for tracking one or more surgical instruments 200 including the surgical instrument 200 and the tracker 300 to assist the medical professional, such as a surgeon, in executing a medical procedure.

The surgical navigation system 102 may comprise a navigation interface that includes one or more display units 104 and one or more user inputs 106. The display unit 104 of the surgical navigation system 102 may be configured to display various prompts or data entry boxes. For example, the display unit 104 may be configured to display a text box or prompt that allows the surgeon to manually enter or select the type of surgical procedure to be performed. The display unit 104 may also be configured to display patient data, such as a pre-operative image or scan. As described above, the pre-operative image may be based on MRI scans, radiological scans, or computed tomography (CT) scans of the patient's anatomy. The preoperative image may be uploaded to the surgical navigation system 102 and displayed on the display unit 104. The display unit 104 may be further configured to display a surgical plan for a medical procedure overlaid on the patient data or image. The surgical plan may include the surgical pathway for executing the medical procedure or planned trajectory or orientation for the medical instrument during the medical procedure. The surgical plan may also include overlaying the position and/or orientation of an implant or medical device to be inserted during the medical procedure on the patient data or image. It is contemplated that the surgical navigation system 102 may comprise a display unit 104 configured to display and/or project a holographic image of surgical pathway for executing the medical procedure or planned trajectory or orientation for the medical instrument during the medical procedure. This may include projecting the surgical pathway onto the patient or other surface in the operating room. It may also include a projection of the surgical pathway onto the head unit worn by the surgeon, such as a lens, shield, or glasses of the head unit. An exemplary configuration of surgical navigation system 102 including a display unit worn by the surgeon to display the target trajectory and/or target location is disclosed in U.S. Patent Application Pub. No. 2020/0085511.

The user input 106 may be configured to allow the surgeon to input or enter patient data or modify the surgical plan. The patient data may comprise patient images, such as pre-operative images of the patient's anatomy. These images may be based on MRI scans, radiological scans, or computed tomography (CT) scans of the patient's anatomy. The patient data may also include additional information related to the type of medical procedure being performed, the patient's anatomical features, the patient's specific medical condition, and/or operating settings for the surgical navigation settings. For example, in performing a spinal surgery, the surgeon may enter information via the user input 106 related to the specific vertebra on which the medical procedure is being performed. The surgeon may also input various anatomical dimensions related to the vertebrae and/or the size and shape of a medical device or implant to be inserted during the medical procedure. The user input 106 may also be configured to allow the surgeon to select, edit or manipulate the patient data. For example, the surgeon may identify and/or select anatomical features from the patient data. This may include selecting the surgical site, such as selecting the vertebra and/or specific area on the vertebra where the medical procedure is to be performed.

The surgical navigation system 102 may further comprise a navigation processor 108. The navigation processor 108 can be located on a personal computer or laptop computer. The navigation processor 108 may be in communication with the user input 106, display unit 104, central processing unit (CPU) and/or other processors, memory (not shown), and storage (not shown). The navigation processor 108 may further comprise software and/or operating instructions related to the operation of the surgical navigation system 102 and to implement the various routines and/or methods disclosed herein. The software and/or operating instructions may comprise a planning system configured to find an accurate position and/or angular alignment of the surgical instruments 200 in relation to the patient 120. The navigation processor 108 may be in wired or wireless communication with the surgical instrument 200, directly or indirectly.

The surgical navigation system 102 may also comprise a tracking unit, or localizer 122, including one or more sensors 124. The sensors may comprise cameras, such as CCD cameras, CMOS cameras, and/or optical image cameras, magnetic sensors, radio frequency sensors, or any other sensor adapted to detect and/or sense the position of a tracker 300 of the surgical instrument 200. One exemplary localizer 122 is capable of detecting radiation or light from the plurality of markers and of generating a localizer signal representative of the detected radiation or light. An exemplary surgical navigation system 102 may be configured to utilize a tracker 300 with a fixed spatial relation between tracking markers. Description of various suitable localizers that may be utilized can be found in U.S. Patent No. 10,531,926.

The navigation processor 108 may be capable of receiving the localizer signal. The navigation processor 108 may further be capable of registering and tracking the tracker 300 based on the received sensor signal. Based on the localizer signal, the processor is also capable of calculating an orientation and/or position of the tracker 300 relative to the localizer 122. The navigation processor 108 may have access to information about the spatial relation. In such a case, three-dimensional images captured by a stereo camera would not be required and the camera may only comprise a single two-dimensional image sensor.

The navigation processor 108 may further be configured to receive and/or store information/data of a patient 120 (e.g., a computed tomography scan and/or tracking signal of the patient's body). The navigation processor 108 may then calculate a position and/or orientation of the surgical instrument 200 relative to the patient 120. The navigation processor 108 may be configured to generate a visual or acoustic signal indicative of the tracking of the surgical instrument 200. The visual signal may be displayed on the display unit 104. The navigation processor 108 may be a part of a computing device separate from the localizer. Alternatively, the localizer may comprise the navigation processor.

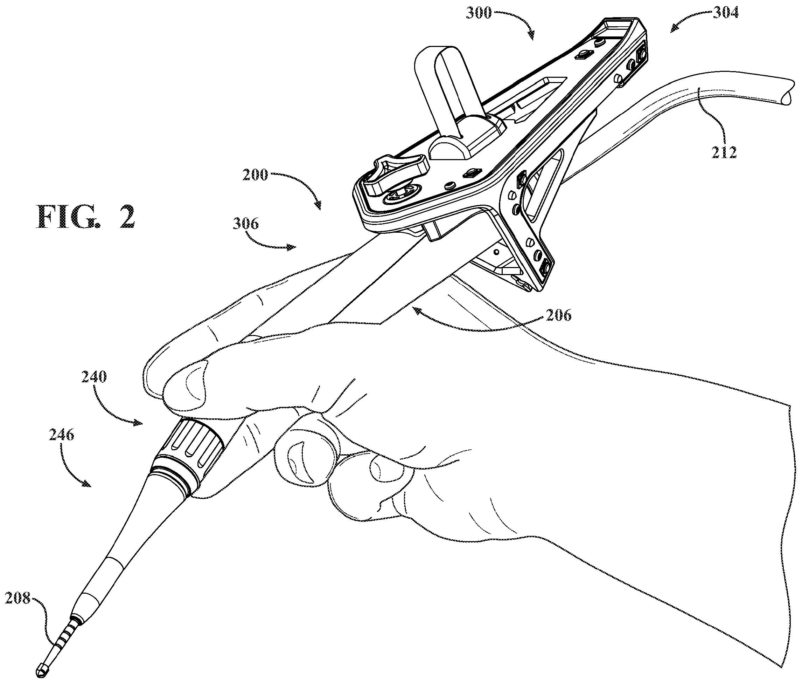

FIG. 2 shows a perspective view of a first configuration of the tracker 300 and the surgical instrument 200. The surgical instrument 200 has a proximal end 202 and a distal end 204 that are spaced along an instrument axis A1. In many cases, such as shown in FIG. 2, the surgical instrument 200 transfers mechanical energy along the instrument axis A1 from a source (e.g., a motor or an ultrasonic transducer) to a cutting tool 208 coupled to the surgical instrument 200. One example of this arrangement is shown in FIG. 2, where the surgical instrument 200 is illustrated with a rotary cutting tool such as a high-speed drill attachment.

The surgical instrument 200 may comprise an attachment 240, a surgical handpiece 206 (or handpiece), and a cutting tool 208. The attachment 240 is configured to engage the surgical handpiece 206 and to receive the cutting tool 208. The attachment 240 transfers rotary motion from the handpiece 206 to the cutting tool 208. As such, the handpiece 206 is powered and receives electrical control signals from, for example, a control system coupled to the handpiece 206 via a flexible supply cable 212.

The handpiece 206 may include a housing 210, a motor (not shown) disposed in the housing 210, a flexible supply cable 212 protruding from the housing 210 in a proximal direction, and a handpiece interface 214 at a distal end 216 of the surgical handpiece 206. The housing 210 has a handpiece diameter 218 Exemplary surgical instruments can be found in U.S. Patent No. 8,597,316 and U.S. Patent No 10,537,339.

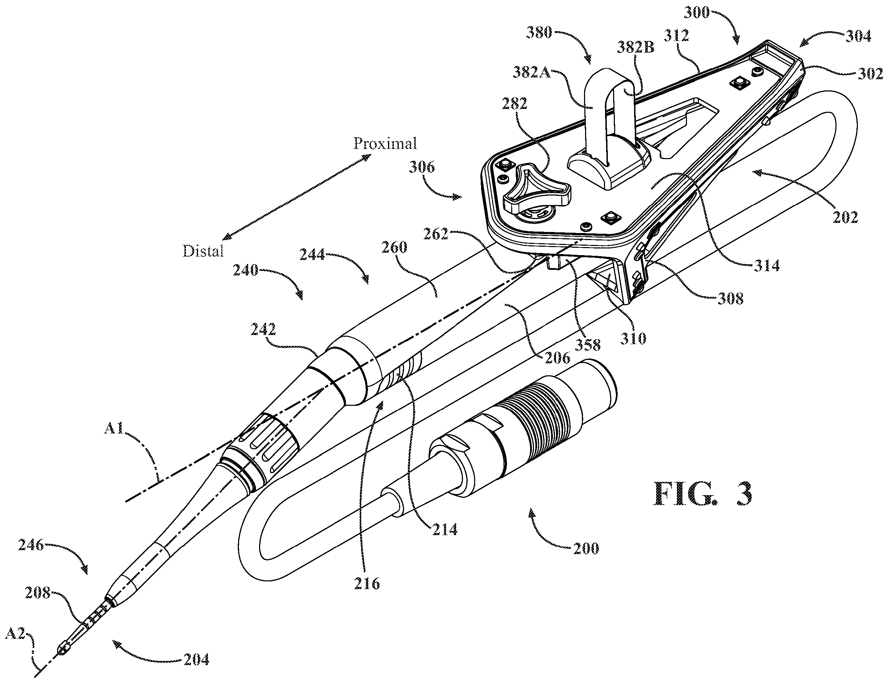

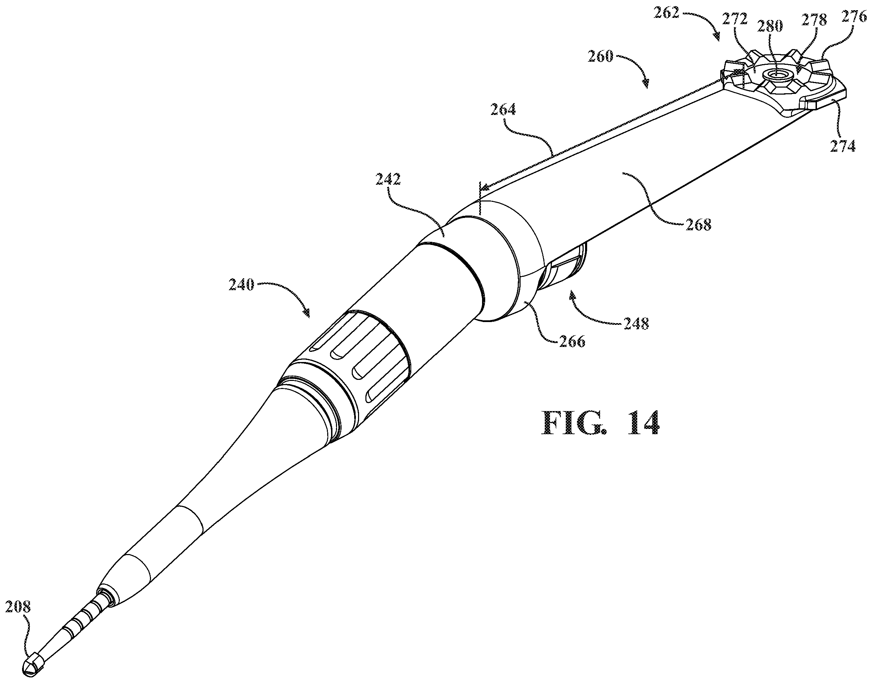

Power from the handpiece 206 is transferred to the attachment 240 to drive the cutting tool 208. Illustrated in FIGs. 3 and 14s, the attachment 240 may comprise an attachment body 242 extending from a proximal end 244 to a distal end 246. As used herein, "distal" generally refers to portions that are closer to the patient during use (e.g., a material removal tool nearest to a surgical site), and "proximal" generally refers to portions that are further from the patient during use (e.g., a power cord). The proximal end 244 of the attachment 240 is configured for engaging the distal end 216 of the surgical handpiece 206. The attachment body 242 comprises a bushing portion 248 arranged at the proximal end 244 of the attachment body 242, which is engageable with the handpiece interface 214 of the handpiece 206. The bushing portion 248 is received by the handpiece interface 214 and is generally aligned with the instrument axis A1. The bushing portion 248 has a first diameter 250 that is sized to fit within the handpiece interface 214 at the distal end 216 of the handpiece 206.

The attachment body 242 further comprises a mount flange 252 arranged distally of the bushing portion 248, which has a second diameter 254. The second diameter 254 of the mount flange 252 is greater than the first diameter 250 of the bushing portion 248. The second diameter 254 is sized so as to limit the insertion of the bushing portion 248 into the handpiece 206. The second diameter 254 may be less than, or equal to, the housing diameter 218 of the handpiece 206. The mount flange 252 abuts the distal end of the handpiece 206 when the attachment 240 is fully coupled to the handpiece 206.

A driveshaft 256 is disposed in the attachment body 242 and supported for rotation about the instrument axis A1 and, in some instances, the tool axis A2. The driveshaft 256 is rotatable about the instrument axis A1 for transferring power from the surgical handpiece 206 to the cutting tool 208. In some implementations of the attachment 240 the driveshaft 256 may comprise two or more driveshaft segments that are engaged in a torque translating relationship such that rotation of one driveshaft segment is transferred to the other driveshaft segment(s). The driveshaft segments may be operably coupled using, for example, a universal joint, a constant velocity joint, gears, etc. To this end, some embodiments of the attachment 240, may be angled relative to the instrument axis A1. The attachment 240 shown herein is such an attachment with the distal end 246 angled relative to the proximal end 244. Here, one of the driveshaft segments 256A is arranged in a proximal portion of the attachment 240 and aligned with the instrument axis A1 and the other driveshaft segment 256B is arranged in a distal portion of the attachment 240 and aligned with the tool axis A2. Angling the attachment 240 may facilitate better control and/or visibility by the surgeon during use. For example, the surgeon may prefer that the working end be angled when performing procedures with one type of cutting tool and prefer that the working end be straight when performing procedures with another type of cutting tool. The attachment 240 could be straight, angled at 15 degrees, 45 degrees, etc. Additionally, the attachment could be of various lengths such as 30 mm, 50 mm, etc.

The attachment 240 further comprises a tool chuck 258 supported in the attachment body 242 and configured to engage the cutting tool 208. The cutting tool 208 is arranged at a working end of the surgical instrument 200. Here, the working end refers generally to a distal-most portion of the surgical instrument 200 intended to perform work (e.g., material removal) on the patient. The tool chuck 258 is operably coupled to the driveshaft 256 to receive power from the handpiece 206. More specifically, the tool chuck 258 may be coupled to a distal driveshaft segment 256B and aligned with a tool axis A2. The tool chuck 258 advantageously allows the cutting tool 208 to be removed from the attachment 240 without first removing other parts of the surgical instrument 200 (e.g., the tracker 300).

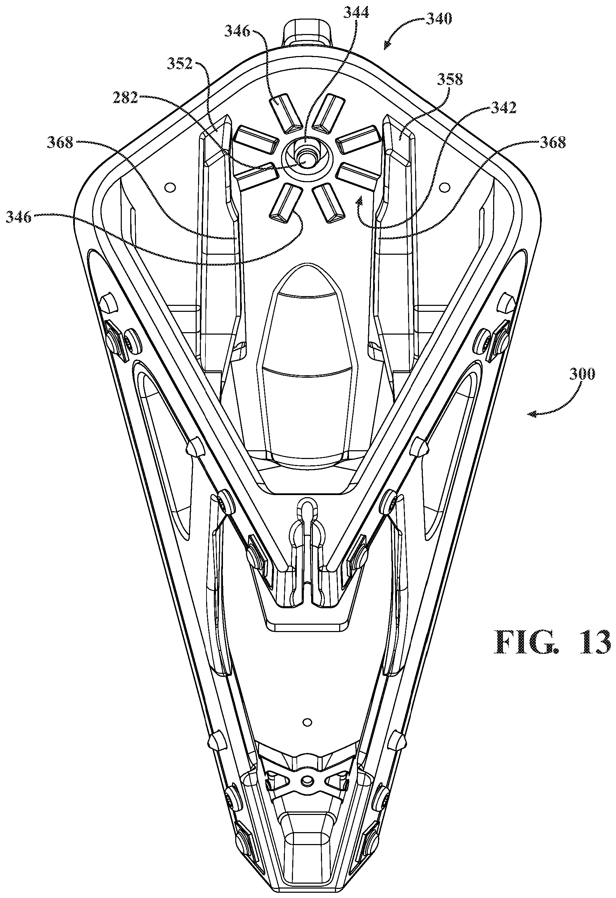

Turning to FIG. 13, in addition to the above, the attachment 240 further comprises a cantilever arm 260. The cantilever arm 260 may be coupled to the attachment body 242 adjacent to, or distal to, the mount flange 252. The cantilever arm 260 extends from the attachment body 242 in a proximal direction to an accessory mount 262. The cantilever arm 260 is configured such that the accessory mount 262 is positioned proximally of the bushing portion 248. More specifically, the accessory mount 262 is spaced from the mount flange 252 at an arm distance 264. An arm socket 266 is arranged at a distal end of the cantilever arm 260 and engages the attachment body 242 for coupling the cantilever arm 260 to the attachment 240. Here, the arm socket 266 is ring shaped and coupled to the attachment body 242 using an interference fit. Said differently, an inner diameter of the arm socket 266 is nearly identical to the second diameter 254 of the bushing portion 248 such that the arm socket 266 may be pressed onto the attachment body 242 and retained without additional fasteners. In other implementations of the attachment body 242 or cantilever arm 260, the arm socket 266 may be welded to the attachment body 242 during manufacturing. It is contemplated that other coupling methods may be utilized, such as brazing, adhesives, soldering, one or more threaded fasteners, staking, swaging, and the like. The arm socket 266 may be removably coupled to both the attachment body 242 and the cantilever arm 260. Alternatively, the arm socket 266 may be integrally formed with the attachment body 242 and the cantilever arm 260.

The cantilever arm 260 extends in a direction that is generally parallel to the instrument axis A1. The cantilever arm 260 is shaped so as to minimize the profile of the surgical instrument 200. Said differently, the cantilever arm 260 is shaped such that an outer surface of the cantilever arm 260 to the instrument axis A1 is minimized. To this end, the cantilever arm 260 may have a curved profile 268 that is spaced from and follows the cylindrical shape of the handpiece 206. More specifically, the cantilever arm has an arm radius distance 270 from the curved profile 268 to the instrument axis A1. A ratio of the handpiece diameter 218 to arm radius distance 270 is greater than 1.25:1, which greatly reduces the footprint of the surgical instrument 200 and improves balance in the surgeon's hand. Similarly, a ratio of the arm distance 260 to the arm radius distance 270 is greater than 5:1.

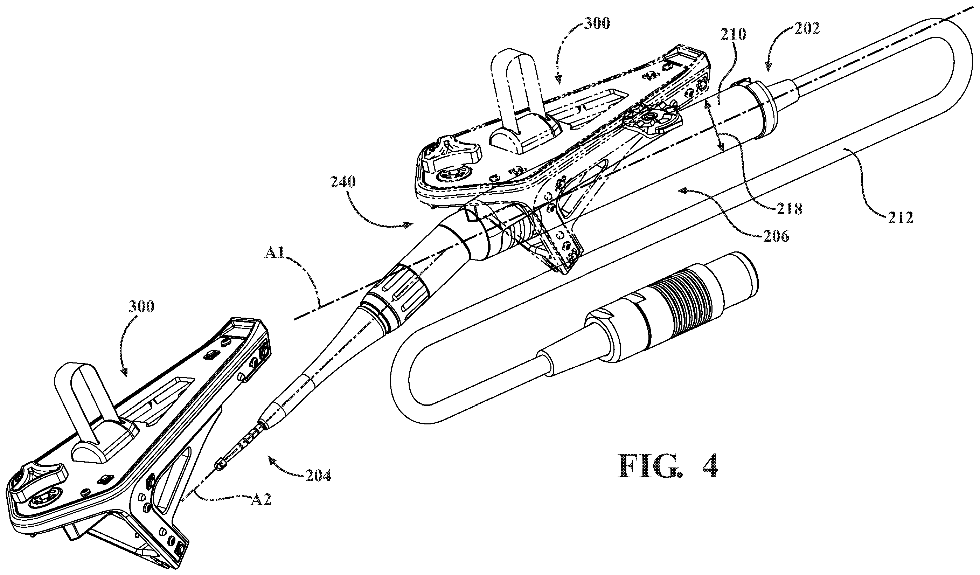

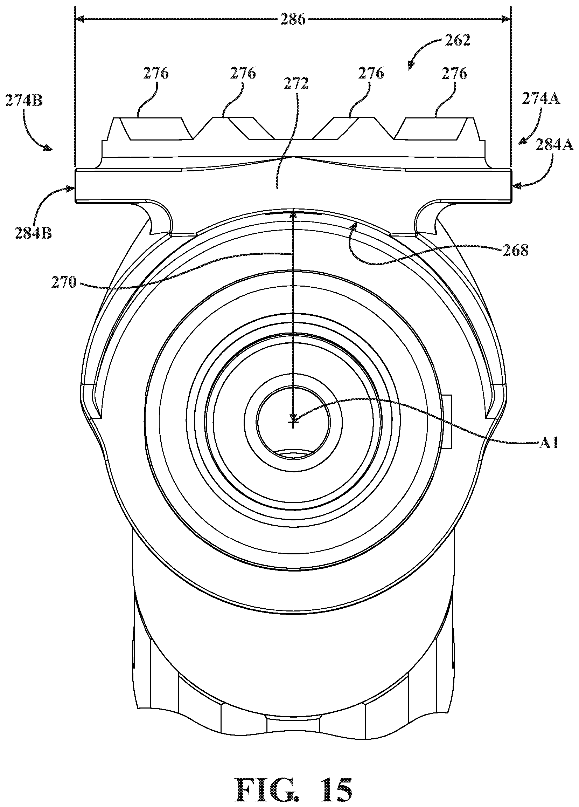

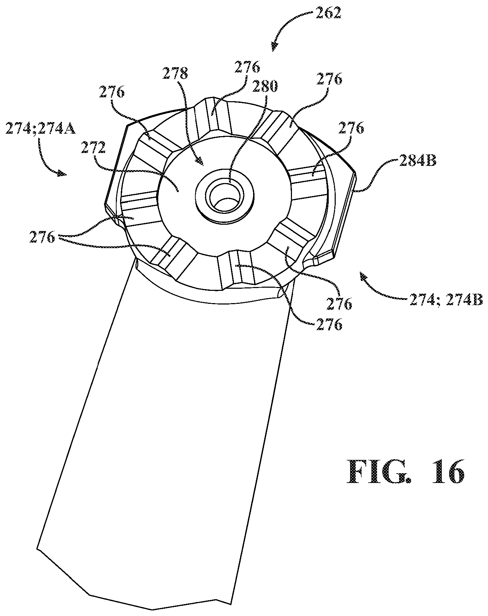

FIGS. 13-16 show the accessory mount 262 coupled to the proximal end of the cantilever arm 260. The accessory mount 262 is configured to engage with an accessory socket 340 (see FIG. 4). As will be discussed below, the accessory socket 340 is coupled to the tracker frame 302 of the tracker 300 for removably coupling the tracker 300 to the attachment 240. The accessory mount 262 may comprise a trunk portion 272, first and second lateral wing portions 274, and one or more alignment pegs 276. The trunk portion 272 may have a top surface 278 and define a clamping hole 280. The top surface 278 is generally oriented facing away from the instrument axis A1. The clamping hole 280 is configured to receive a corresponding clamp fastener 282, discussed below. Here, the clamping hole 280 is threaded with a female thread form to receive the clamp fastener 282 and the clamp fastener 282 is threaded with a correspondingly shaped male thread form.

The first and second lateral wing portions 274 may be further defined as a first lateral wing portion 274A and a second lateral wing portion 274B. The first lateral wing portion 274A and the second lateral wing portion 274B protrude from opposing sides of the trunk portion 272 in a direction that may be generally transverse to the top surface 278. Each of the lateral wing portions 274 has a corresponding lateral face 284. More specifically, the first lateral wing portion 274A has a first lateral face 284A and the second lateral wing portion 274B has a second lateral face 284B. As illustrated in the example of FIG. 14, the lateral faces 284 are, in general, at an approximately 90-degree angle to the top surface 278. However, some variance is expected due to the nature of certain manufacturing processes. In other implementations the angle of the lateral faces 284 and the top surface 278 may be any value between 0 and 180 degrees. The lateral faces 284 are, however, non-parallel with the instrument axis A1. A mount width 286 may be defined between the first lateral face 284A and the second lateral face 284B.

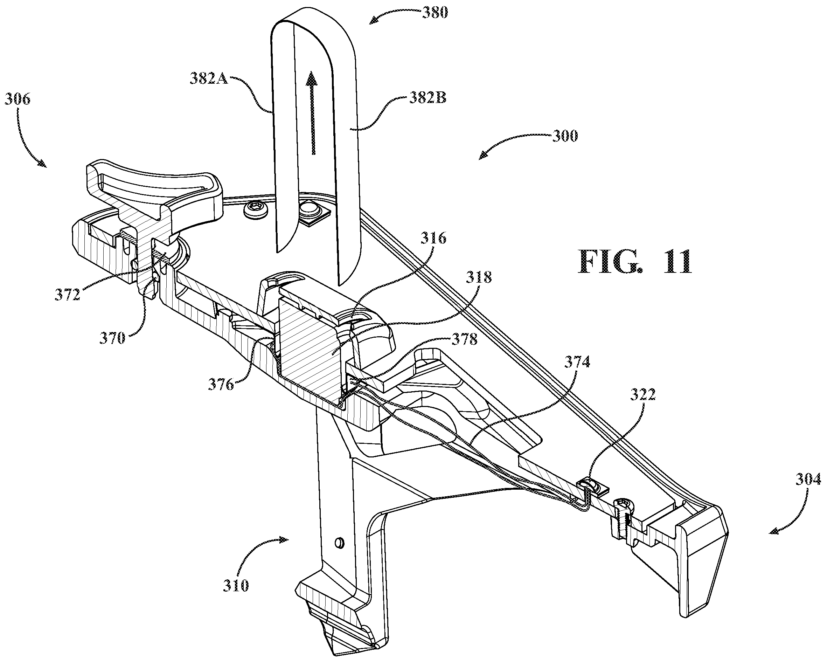

A longitudinal plane P1 (see FIG. 11) aligned with the instrument axis A1 and arranged in a vertical orientation is defined by the surgical instrument 200. The longitudinal plane P1 illustratively defines left and right sides of the surgical instrument 200. The first and second lateral faces 284 may be symmetrically angled about the longitudinal plane P1 of the surgical instrument 200. Said differently, the first lateral face 284A may be at a first angle relative to the longitudinal plane P1 and the second lateral face 284B may be at a second angle relative to the longitudinal plane P1. The first angle of the first lateral face 284A and the second angle of the second lateral face 284B may be equal and opposite to each other i.e., mirrored across the longitudinal plane P1. The exemplary accessory mount 262 shown here implements lateral faces 284 that would, illustratively, intersect at a point that is spaced from the accessory mount 262 in a distal direction.

The accessory mount 262 may further comprise the one or more alignment pegs 276 protruding from the top surface 278. The alignment pegs 276 are configured for engagement with corresponding alignment channels 346 defined in the accessory socket 340. The accessory mount 262 may comprise any suitable number of alignment pegs 276 so as to effect secure engagement with the accessory socket 340. More specifically, the alignment pegs 276 prevent movement of the tracker 300 relative to the attachment 240 during use and, as such, relative movement therebetween may reduce the tracking accuracy. The one or more alignment pegs 276 may further be defined as eight alignment pegs, such as illustrated herein. Other quantities of alignment pegs are also contemplated.

Here, the alignment pegs 276 may be utilized to limit relative movement between the tracker 300 and the accessory mount 262. The alignment pegs 276 may be radially arranged about the clamping hole 280 in a circular, or semi-circular, pattern that limits movement. Here, the alignment pegs 276 are shaped with an elongated and truncated pyramid shape. A mount height 288 may be defined between a lowermost surface of the lateral wings 274 and an uppermost point of the alignment pegs 276.

As mentioned above, the tracker 300 may be removably coupled to the surgical instrument 200 in order to track the position and orientation of the attachment 240 and the cutting tool 208. The tracker 300 is operable with the surgical navigation system 102 to determine the position and orientation of the tracker 300 in space. The tracker 300 is operable with the surgical instrument 200 and the surgical navigation system 102 to determine the position and/or orientation of the surgical instrument 200 or a component thereof within an operating room. In order to accurately determine the position of the surgical instrument 200, the tracker 300 is coupled to the surgical instrument 200 and configured to prevent relative movement therebetween during a surgical procedure. Additionally, the tracker 300 should be coupled to the surgical instrument 200 so as to maximize visibility of the tracker 300 by the surgical navigation system 102. It should also be appreciated that certain features of the described trackers may be used to track a patient, another device in the operating room, or even a medical professional.

While the surgical instrument 200 is shown throughout the figures as a high-speed drill, the tracker 300 may be utilized with surgical instruments other than high-speed drills. For example, the tracker 300 could be coupled to a handheld ultrasonic ablation tool, a biopsy needle, or a portion of a robotic device such as robotic end effector, a robotic arm, or other device in the operating room. Similarly, the tracker 300 could be adapted to couple to other surgical instruments (not shown) such as a handheld drill, a saw, or a bur. As mentioned above, the attachment 240 coupled to the working end of the surgical instrument 200 is shown in FIG. 2 as an angled attachment, which drives a rotary tool on the tool axis A2 different than the instrument axis A1. For example, the attachment could be straight, angled at 15 degrees, 45 degrees, etc.; the attachment could be of various lengths such as 30 mm, 50 mm, etc.

In some cases, some surgical procedures may require the surgeon to perform precise movements with the surgical instrument. These and other surgical procedures may span a duration of several hours. If a heavy tracker is attached to a handheld surgical instrument, the tracker may shift the center of mass of the assembly, which can be tiresome for the surgeon (e.g., due to a torque applied at the holding hand). The tracker 300 illustrated throughout the figures may offer a reduced weight due to material selection and design features that minimize the amount of material required to maintain a shape and desired characteristics of the tracking device. The handheld surgical instrument 200 may be less tiresome to hold.

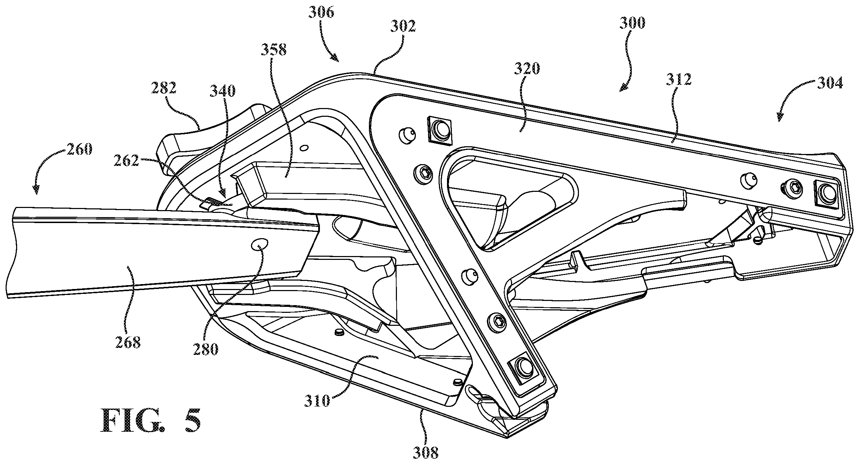

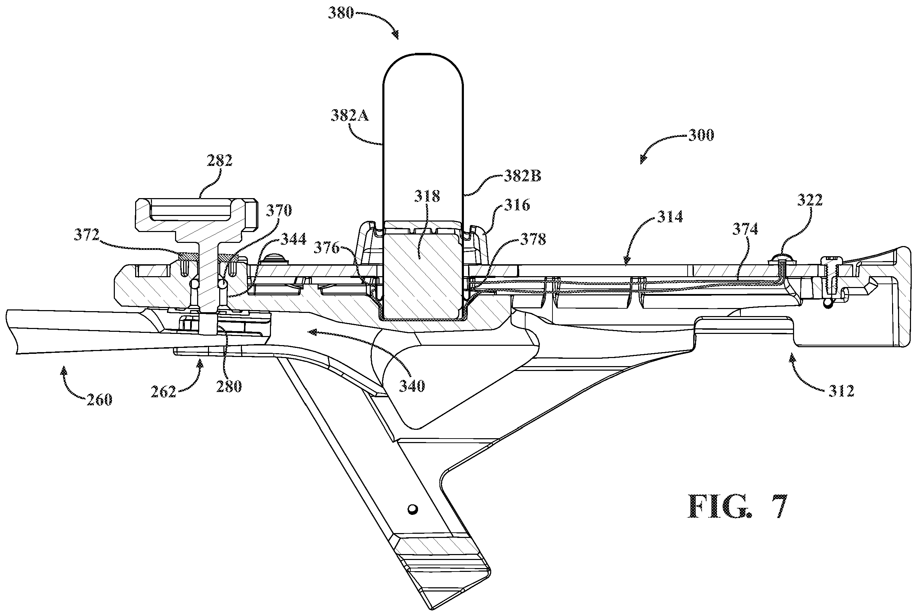

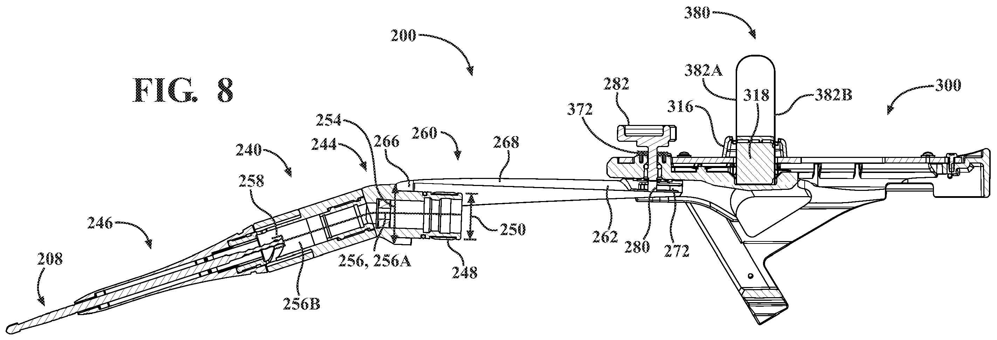

Turning now to FIGs. 2-12, the first implementation of the tracker 300 is shown. In order to facilitate removably coupling the tracker 300 to the surgical instrument 200, the tracker 300 comprises a tracker frame 302 having a proximal end 304 and a distal end 306, as illustrated in FIG. 4. The tracker frame 302 comprises a mounting portion 308 and defines an instrument aperture 310 through which the attachment 240 is inserted to couple to the tracker 300. As will be discussed in further detail below, the surgical instrument 200 engages with the tracker 300 from the proximal end 304 of the tracker frame 302. As shown in FIG. 3, the tracker 300 is shown being removed/assembled to the attachment 240. A first tracker 300 is shown in phantom in a partially assembled state with the attachment 240 arranged in the instrument aperture 310 and a second tracker 300 is shown spaced from the attachment 240. The tracker 300 is assembled to the attachment 240 by inserting the distal end of the attachment 240 through the instrument aperture 310 from a proximal end 304 of the tracker 300. The instrument aperture 310 is shaped and sized so as to stop the tracker 300 once the assembled position is reached and not fall off the proximal end of the surgical instrument 200.

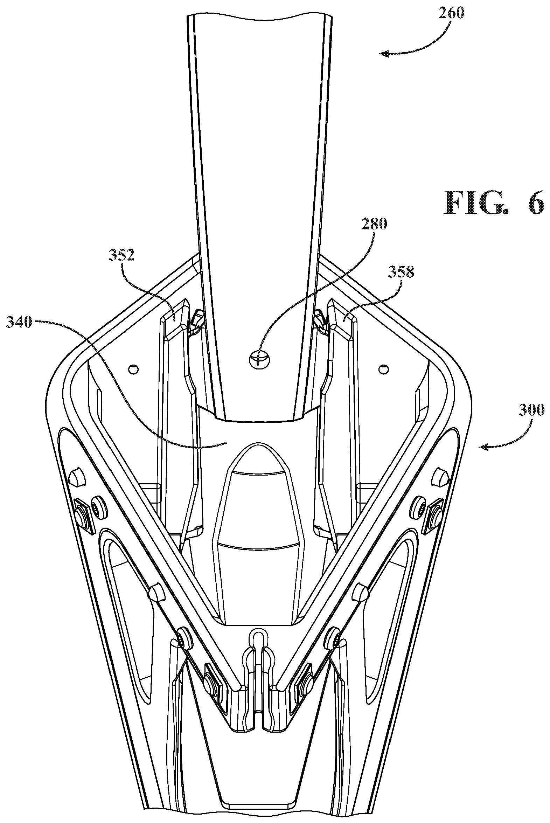

Turning to FIG. 6, the tracker frame 302 may further comprise an elongated portion 312 supported on the mounting portion 308 and extending proximally and in a direction generally parallel to the instrument axis A1. The elongated portion 312 may have a top face 314. The top face 314 may have a generally triangular shape when viewed from above, and the elongated portion 312 may taper from the distal end 306 to the proximal end 304. The tracker frame 302 may define a battery receptacle 316 arranged adjacent to the top face 314 of the tracker frame 302. The battery receptacle 316 may be configured to receive a battery 318 in order to place the battery 318 in electrical communication with an electrical circuit 374 of the tracker 300. The battery receptacle 316 may be configured to prevent current flow from the battery 318 until the battery 318 has been fully inserted into the battery receptacle 316. One exemplary implementation of LED tracking markers is shown in U.S. Patent Application Pub. No. 2019/0321108. Other implementations are contemplated.

In order to track the position and orientation of the surgical instrument 200, the tracker 300 may further comprise a tracker array 320 having a plurality of tracking markers 322 optionally arranged on one or more tracked faces 324A, 324B, 324C and coupled to the tracker frame 302, as shown in FIG. 11. A first tracked face 324A is arranged on the top face 314 of the elongated portion 312, a second tracked face 324B is arranged beside the elongated portion 312 and the mounting portion 308 on the first side of the longitudinal plane P1, and a third tracked face 324C is arranged beside the elongated portion 312 and the mounting portion 308 on the second side of the longitudinal plane P1. The tracker array 320 may further have a tracker profile defined perpendicular to the instrument axis A1 comprising three radial segments. The tracked faces 324A, 324B, 324C may be aligned with a respective radial segment 326A, 326B, 326C, each side oriented non-parallel to the others, for example approximately 120 degrees. The plurality of tracked faces 324A, 324B, 324C are positioned such that each tracked face 324A, 324B, 324C is oriented in a different direction from each other. The first tracked face 324A may be aligned with the first radial segment 326A, the second tracked face 324B may be aligned with the second radial segment 326B, and the third tracked face 324C may be aligned with the third radial segment 326C. Other number of arrays and/or number of sides are also contemplated. A tracker utilizing a similar arrangement of tracked faces is disclosed in U.S. Patent Application Pub. No. 2021/0236212.

As shown in FIGS. 4 and 11, at least one of the tracking markers 322 may be positioned on the elongated portion 312 distally of the mounting portion 308 and at least another one of the tracking markers 322 may be positioned proximally of the mounting portion 308. In some implementations, such as shown here, the tracker array 320 may comprise at least three tracking markers 322, wherein the at least three tracking markers 322 define a marker plane on one of the tracked faces 324A, 324B, 324C. At least one of the tracking markers 322 may be arranged in each of the radial segments 326A, 326B, 326C to facilitate increased visibility to the navigation system.

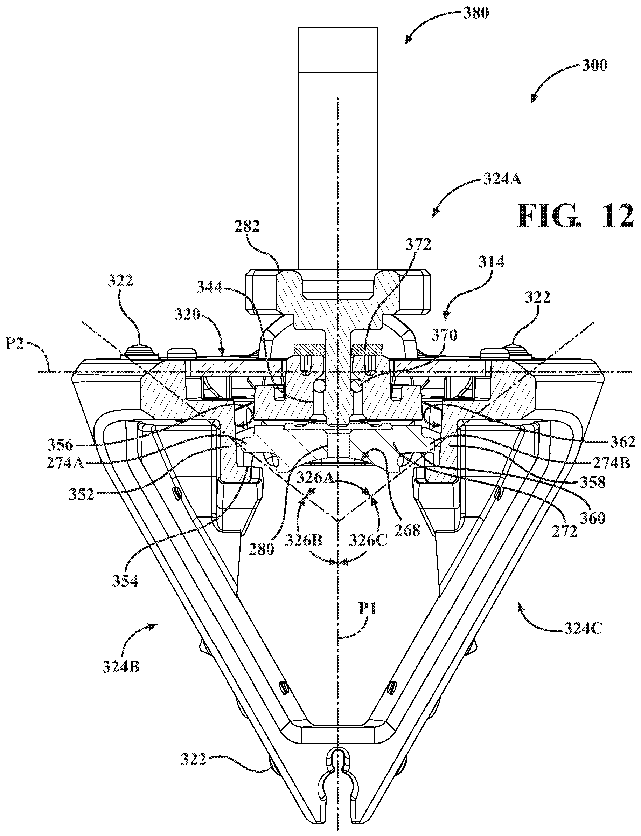

As mentioned above, the tracker 300 may be clamped to the surgical instrument 200 in a secure and reliable manner. To this end, the tracker 300, as shown in FIG. 12, may further comprise an accessory socket 340, which is operable to effect attachment of the tracker 300 to the surgical instrument 200. The accessory socket 340 is removably couplable to the accessory mount 262 by the surgeon or another user or other medical professional. The accessory mount 262 coupled to the surgical instrument 200 and the accessory socket 340 coupled to the tracker 300 may collectively be referred to as a coupling arrangement.

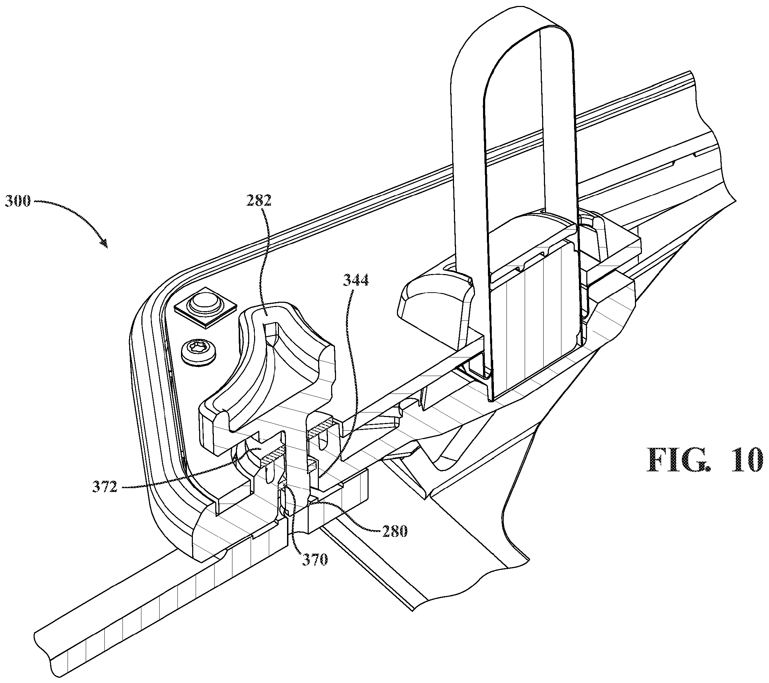

With reference to FIGS. 10-12, the accessory socket 340 may comprise a clamp surface 342 defining a fastener hole 344, and one or more alignment channels 346 recessed in the clamp surface 342. The alignment channels 346 are configured to complementarily engage the one or more alignment pegs 276 of the accessory mount 262. The accessory socket 340 may further define a socket opening 348, which is sized and shaped to receive the accessory mount 262. The socket opening 348 is generally arranged at a proximal end of the accessory socket 340.

The accessory socket 340 further comprises a distal wall 350 adjacent to the clamp surface 342. The distal wall 350 is arranged at the distal end 306 of the tracker frame 302 opposite the socket opening 348. The distal wall 350 protrudes away from the clamp surface in a direction toward the surgical instrument 200. When the accessory mount 262 is received in the accessory socket 340 the distal wall 350 limits movement of the accessory mount 262 in a proximal to distal direction. Said differently, accessory socket 340 receives the accessory mount 262 in the socket opening 348 at the proximal end. As the accessory mount 262 moves in a proximal to distal direction the distal wall 350 limits movement and retains the accessory mount 262 in the accessory socket 340, when the accessory mount 262 engages the distal wall 350 the accessory mount 262 is in a fully inserted position. The distal wall 350 prevents the tracker frame 302 from becoming disengaged from the accessory mount 262 before the clamp fastener 282 can be tightened.

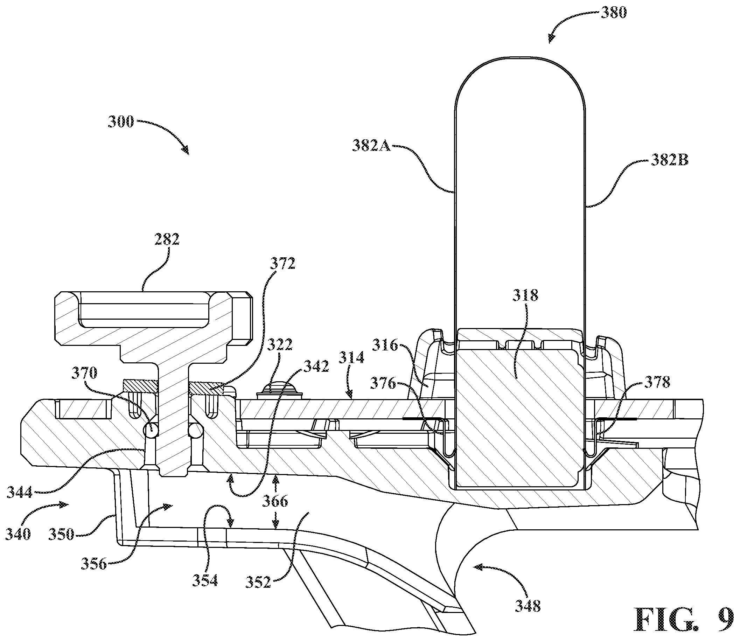

The accessory socket 340 may further comprise first and second socket walls extending from the clamp surface 342. More specifically, the first socket wall 352 and the second socket wall 358 each extend from the clamp surface 342, as shown in FIG. 9. The first socket wall 352 may have a corresponding first rail surface 354 spaced from the clamp surface 342. The first socket wall 352 may further have a first ramp surface 356 extending between the first rail surface 354 and the clamp surface 342. The second socket wall 358 may have a corresponding second rail surface 360 spaced from the clamp surface 342. The second socket wall 358 may further have a second ramp surface 362 extending between the second rail surface 360 and the clamp surface 342. The first socket wall 352, first rail surface 354, and first ramp surface 356 are arranged on one side of the longitudinal plane P1 to partially define the accessory socket 340. The second socket wall 358, the second socket rail surface 360, and the second ramp surface 362 are arranged across the longitudinal plane P1 to partially define the accessory socket 340. The first socket wall 352, the second socket wall 358, and the distal wall 350 cooperate to define the accessory socket 340.

The accessory socket 340 defines a socket width 364 and a socket height 366. The socket width 364 may be defined as the distance between the first ramp surface 356 of the first socket wall 352 and the second ramp surface 362 of the second socket wall 358. The socket height 366 may be defined as a distance between the clamp surface 342 and the first rail surface 354. The distance between the clamp surface 342 and the second rail surface 360 is, in general, equal to the distance between the clamp surface 342 and the first rail surface 354. In other implementations in which the distance between the clamp surface 342 and the second rail surface 360 differs from the distance between the clamp surface 342 and the first rail surface 354, the distance between the clamp surface 342 and the second rail surface 360 may alternatively define the socket height.

Similar to the lateral faces 284, the first ramp surface 356 and the second ramp surface 362 are, in general, at an approximately 90-degree angle to the clamp surface 342. However, some variance is expected due to the nature of certain manufacturing processes. The ramp surfaces are, however, non-parallel with the instrument axis A1. The first ramp surface 356 and the second ramp surface 362 may be symmetrically angled about the longitudinal plane P1 of the surgical instrument 200. Said differently, the first ramp surface 356 may be at a first angle relative to the longitudinal plane P1 and the second ramp surface 362 may be at a second angle relative to the longitudinal plane P1. The first angle of the first ramp surface 356 and the second angle of the second ramp surface 362 may be equal and opposite to each other. Furthermore, the angle of the first socket wall 352 is the same as an angle of the first lateral face 284A of the first lateral wing portion 274A relative to the longitudinal plane P1 of the surgical instrument 200 and the angle of the second socket wall 358 is the same as the angle of the second lateral face 284B of the second lateral wing portion 274B relative to the longitudinal plane P1 of the surgical instrument 200. In this way, engagement between the first ramp surface 356 and the first lateral face 284A is facilitated. Similarly, engagement between the second ramp surface 362 and the second lateral face 284B is facilitated. The mount width 286 of the accessory mount 262 between the first lateral face 284A and the second lateral face 284B is larger than the socket width 364 of the accessory socket 340 such that the tracker 300 cannot be slid off the back of the accessory mount 262 and the surgical instrument 200. Similarly, the mount height 288 of the accessory mount 262 between the between a lowermost surface of the lateral wings 274 and the uppermost point of the alignment pegs 276 is less than the socket height 366. Said differently, the socket height 366 may be greater than the mount height 288.

Turning now to FIGS. 9 and 10, a channel opening 368 may be defined in at least one of the first rail surface 354 and the second rail surface 360. The channel opening 368 may be a hole or a notch defined in at least one of the first rail surface 354 and the second rail surface 360 and aligned with one or more of the alignment channels 346 defined in the clamp surface 342 of the accessory socket 340. The channel opening 368 may be further defined as one or more channel openings 368, with each of the channel openings 368 aligned with one or more of the alignment channels 346. When the channel openings 368 are aligned in the first rail surface 354 and the second rail surface 360 with the alignment channels 346, a user is afforded improved access to the alignment channels 346, which may facilitate inspection (e.g., to identify damage), cleaning, as well as during manufacturing of the tracker 300.

As mentioned above, the clamp fastener 282 is disposed in the fastener hole 344 and engageable with the clamping hole 280 of the accessory mount 262. The clamp fastener 282 is configured to urge the top surface 278 of the trunk portion 272 toward the clamp surface 342 of the accessory socket 340. When the clamp fastener 282 is received in the clamping hole 280 and tightened, the clamp fastener 282 exerts a compression force on the accessory mount 262 against the accessory socket 340 that urges the alignment pegs 276 into engagement with the alignment channels 346. Said differently, the clamp fastener 282 draws the top surface 278 toward the clamp surface 342 such that the alignment pegs 276 are urged into the alignment channels 346. Generally, the alignment pegs 276 are wider than the alignment channels 346 so as to promote repeatable alignment when engaged. Further, when the alignment pegs 276 are wider than the alignment channels 346, the alignment pegs 276 reach full insertion into the alignment channels 346 before the top surface 278 engages the clamp surface 342. The clamp fastener 282 is arranged near a distal end of the tracker 300 so as to position the tracker frame 302 proximally of the accessory mount 262. As such, the tracking markers 322 are arranged proximally of the clamp fastener 282 and are arranged proximally of the accessory mount 262 when the accessory socket 340 is engaged with the accessory mount 262.

Ease of assembly of the tracker 300 to the attachment 240 is improved by reducing the radial clearance between the clamp fastener 282 and the fastener hole 344 as well as the length of the clamp fastener 282. By reducing the radial clearance, wobbling of the clamp fastener 282 in the fastener hole 344 is reduced, which allows the clamp fastener 282 to easily slide within the fastener hole 344 as the tracker 300 is assembled on to the accessory mount 262. Additionally, the clamp fastener 282 may be captively retained in the fastener hole 344. A captive fastener prevents the clamp fastener 282 from being easily removed from the fastener hole 344 and may further reduce wobble of the clamp fastener 282. In one exemplary implementation shown here, the accessory socket may further comprise a captured washer 370 arranged around a groove in the clamp fastener 282 at a height positioned within the fastener hole 344. The captured washer 370 retains the clamp fastener 282 in the fastener hole 344 to aid a caregiver in assembling the tracker 300 to the accessory mount 262. The captured washer 370 may be formed from a polymer material such as polyether ether ketone (PEEK). Other materials are contemplated. Other implementations are contemplated, such as a spring, wave washer, and a rubber O-ring. For example, an O-ring may be arranged around the clamp fastener 282 and in the fastener hole 344 to captively retain the clamp fastener 282 in the fastener hole 344. The O-ring may be formed from a rubber material (for example, Buna-N, Viton fluoroelastomer, silicone, EPDM, and the like) that grips an interior surface of the fastener hole 344 and the clamp fastener 282 to increase the force required to move the clamp fastener 282. The O-ring may further hold the clamp fastener 282 in a retracted position with the threaded end recessed below the clamp surface 342.

Once the tracker 300 has been assembled to the attachment 240 and the clamp fastener 282 tightened, the coupling arrangement may be subject to strong and/or high-frequency vibrations during use. In order to prevent these vibrations from causing the clamp fastener 282 to loosen, a second washer 372 may be arranged adjacent to the fastener hole 344 to receive the clamp fastener 282. The second washer 372 may be disposed between a head portion of the clamp fastener 282 and the tracker 300. Similar to the captured washer 370, the second washer 372 may be formed from a polymer material, such as polyether ether ketone (PEEK) that grips the head portion of the clamp fastener 282 to increase the force required to loosen the clamp fastener 282, and therefore prevent relative movement between the clamp fastener 282 and the fastener hole 344. Other materials are contemplated. Other materials are contemplated such as Buna-N, Viton fluoroelastomer, silicone, EPDM, neoprene, and other rubber materials. Other implementations are contemplated, such as a spring, wave washer, and the like.

Similar to the alignment pegs 276 described above, the accessory socket 340 may comprise any suitable number of alignment channels 346 so as to effect secure engagement with the accessory mount 262. The one or more alignment channels 346 may further be defined as eight alignment channels, such as illustrated herein. Other quantities of alignment channels are also contemplated. The alignment channels 346 may be radially arranged about the fastener hole 344 in a circular pattern that limits rotational movement between the accessory socket 340 and the accessory mount 262. Here, the alignment channels 346 are shaped with an elongated pyramid shape.

In addition to the longitudinal plane P1 described above, a tracker plane P2 is defined by the tracker 300. More specifically, the tracker plane P2 is defined by at least three of the markers 322. To this end, the tracker frame 302 may be arranged between the accessory socket 340 and the tracker plane P2, or said differently, the tracker frame 302 may be arranged between the accessory socket 340 and the markers 322. Additionally, the tracker 300 may be coupled to, and arranged such that, the tracker plane P2 is parallel to the instrument axis A1.

The various implementations of the tracker 300 shown herein may comprise a tracker frame 302 formed from a material that may comprise a metal such as titanium, a polymer such as nylon, or an epoxy resin such as aromatic epoxy amine resin. The tracker frame 302 may comprise any other suitable materials for use in a medical setting and providing the necessary rigid structure to the tracker 300. The tracker frame 302 may be formed using an injection molding or additive manufacturing process, which forms the tracker frame 302 as a single unitary body. By forming the tracker frame 302 as a single unitary body certain manufacturing steps may be eliminated. Furthermore, dimensional accuracy of the tracker frame 302 may be increased by reducing tolerance stack-ups. In addition, by eliminating any joints between pieces, the stiffness of the tracker frame 302 may be increased. Geometry that would be formed for the purpose of joining multiple pieces together may be eliminated further reducing the weight of the tracker frame 302 and the need to control the accuracy of mating surfaces. In another exemplary implementation of the tracker, the tracker frame 302 be formed using a stereolithography process and an epoxy resin. Alternatively, the tracker frame 302 may be assembled using two or more independent pieces that have been formed via an injection molding process, a blow molding process, a rotary molding process, and the like. The pieces of the tracker frame 302 may be assembled using an adhesive such as epoxy, fasteners, interlocking assembly, heat staking, and combinations thereof.

Previously mentioned above, the tracker 300 may further comprise the tracker array 320 having the plurality of tracking markers 322. The tracking markers 322 may be implemented as LED emitters, also described above. As such, the tracking markers 322 may be configured to receive electrical power from the battery 318. The tracker 300 may comprise an electrical circuit 374 to place the tracking markers 322 and the battery 318 in electrical communication. The electrical circuit 374 may comprise one or more printed circuit boards, wires, connectors, and/or other conductors. The electrical circuit 374 may further comprise a positive terminal 376 and a negative terminal 378 each arranged in the battery receptacle 316, as shown in FIG. 6. The electrical circuit 374 facilitates electrical communication between the positive terminal 376 and the negative terminal 378 and the tracking markers 322.

Each of the positive terminal 376 and the negative terminal 378 are arranged so as to engage corresponding positive and negative terminals (not shown) of the battery 318. Prior to the procedure in which the tracker 300 will be used, the tracker 300 may be subjected to one or more sterilization processes to remove all contaminants from the tracker 300. In some cases, the tracker 300 may be sterilized in its fully assembled state, which includes the battery 318 disposed in the battery receptacle 316. Following the sterilization process the tracker 300 may be stored for an unknown amount of time prior to use. In order to prevent the battery 318 from being discharged during this storage period the tracker 300 may further comprise an isolating strip 3820 disposed between the battery 318 and each of the positive terminal 376 and the negative terminal 378 of the electrical circuit, which prevents the flow of electricity to the tracking markers 322. The isolating strip 380 is lightweight and inexpensive, which allows the electrical circuit 374 to be free from a switch, further reducing costs of the tracker 300.

At the beginning of a tracked surgical procedure, the surgeon or other medical professional activates the tracker 300 by removing the isolating strip 380 from the tracker 300 to enable electrical communication between the tracking markers 322 and the battery 318. Said differently, removing the isolating strip 380 from the tracker 300 allows electricity to flow between each of the positive terminal 376 and the negative terminal 378 and the respective terminal on the battery 318.

In the implementation shown here, the isolating strip 380 comprises a first isolating member 382A and a second isolating member 382B that are joined so as to form a single isolating strip 380 extending between the positive terminal 376 and the negative terminal 378. Joining the isolating members 382A, 382B into the single isolating strip 380 allows the user to remove both isolating members 382A, 382B simultaneously. It is contemplated that the first isolating member 382A and the second isolating member 382B may be discrete isolating strips. The first and second isolating members 382A, 382B may be formed using a non-conductive polymer, Mylar, or Kapton material. Other materials are contemplated.

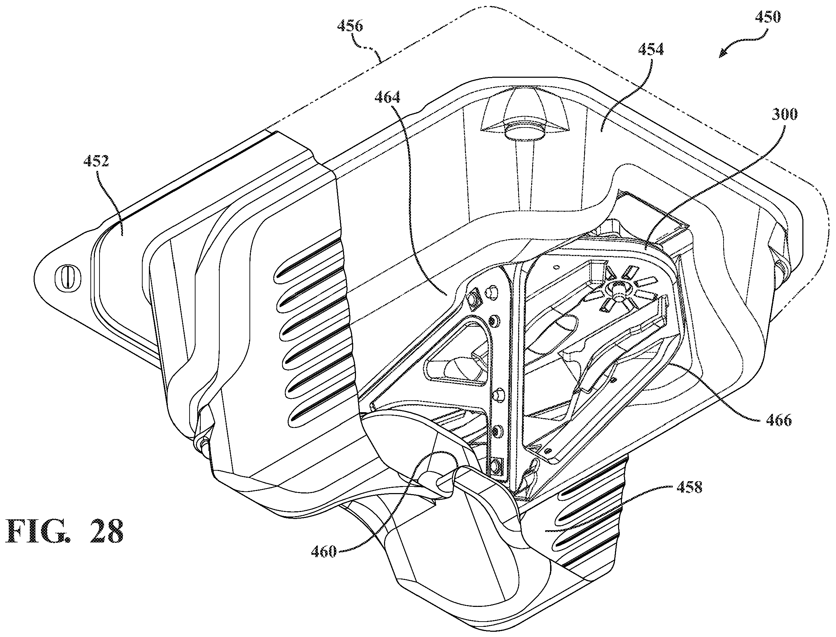

In order to maintain the sterility of the tracker 300 during the aforementioned storage period, the tracker 300 is packaged in a sterile packaging system 450, illustrated in FIGS. 28-31. The sterile packaging system 450 allows the tracker 300 to be transported and stored in non-sterile environments while maintaining sterility. The sterile packaging system 450 may comprise a shell portion 452, an insert portion 454, and a sealing film 456. The shell portion 452, the insert portion 454, and the sealing film 456 may form a blister pack.

The shell portion 452 may define a sterile interior 458 shaped to receive the tracker 300 for sterilization. The sterile interior 458 may comprise one or more protrusions 460 that support the tracker 300 in a particular orientation. For example, the tracker 300 is shown in an upright position with the elongated portion 312 and top face 314 generally facing away from (or toward an opening of) the shell portion 452. The opening of the shell portion 452 is partially defined by an opening face 462, which is a generally planar face surrounding the sterile interior 458. The sealing film 456 is disposed over the sterile interior 458 and cooperates with the opening face 462 to seal the tracker 300 within the sterile packaging system 450. The sealing film 456 engages the opening face 462 and prevents the ingress of foreign material into the sterile interior 458.

The sealing film 456 may be a gas permeable film, such as the type used for ethylene oxide sterilization. The sealing film 456 may be formed of a high-density polyethylene material, such as Tyvek (trademark DuPont). The sealing film 456 may be sealed to opening face 462 of the shell portion 452 using an adhesive, melt sealing, ultrasonic sealing, and the like.

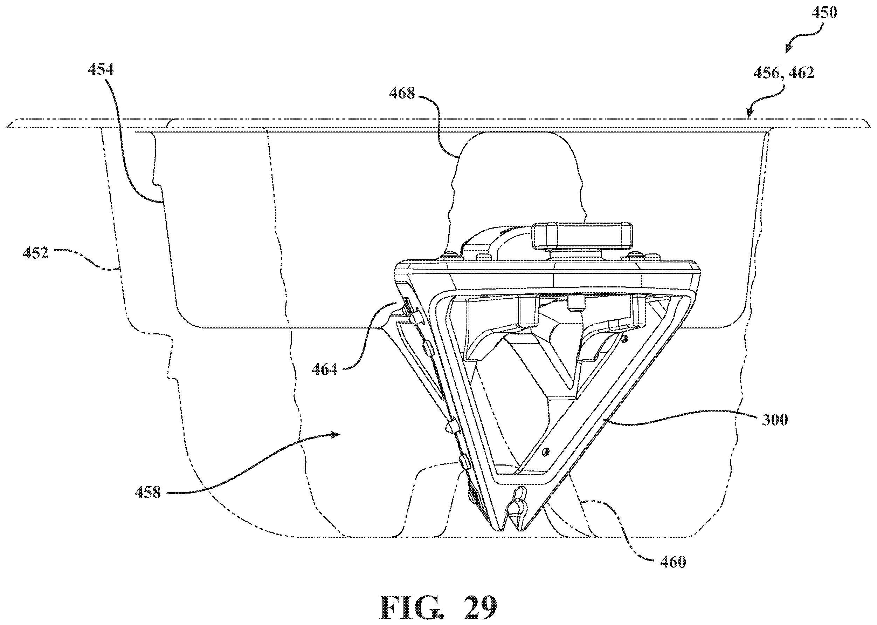

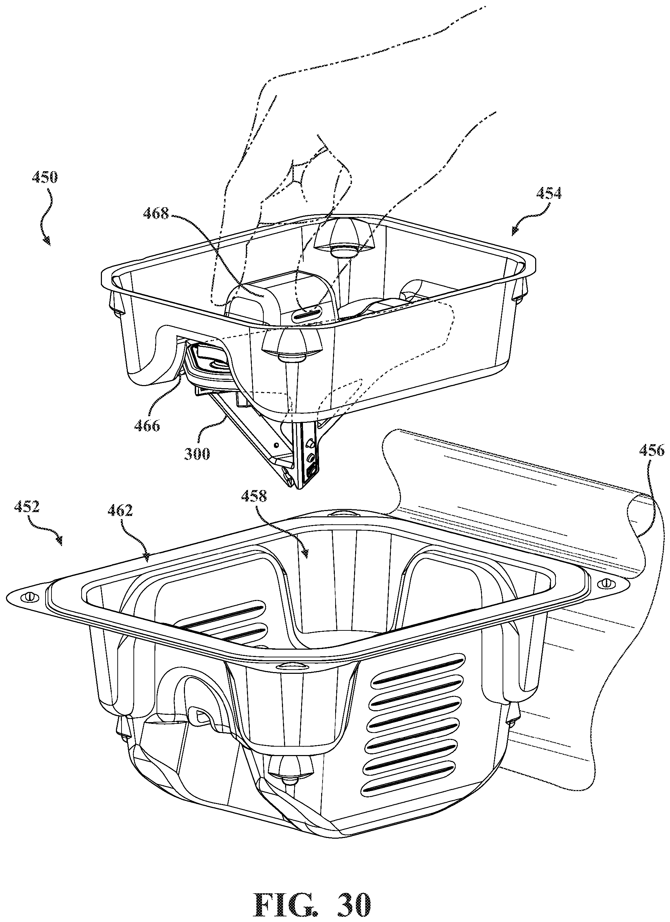

Best shown in FIGS. 29 and 30, the insert portion 454 of the sterile packaging system 450 may comprise one or more undercuts 464 configured for engagement with the tracker 300. The insert portion 454 is positioned in the sterile interior 458 with the tracker 300 arranged between the insert portion 454 and the shell portion 452. The undercuts 464 are areas of the insert portion 454 defined by a profile geometry that would capture a manufacturing mold preventing demolding. The undercuts 464 are arranged on the insert portion 454 to define a cavity 466 shaped to receive a portion of the tracker 300. Here, the cavity 466 is configured to receive the elongated portion 312 of the tracker 300. The undercuts 464 generally match the angled shape of the tracker 300 such that when the elongated portion 312 is inserted into the cavity 466, a width of the tracker 300 is greater than a width between opposing undercuts 464. Because the width between opposing undercuts 464 is less than the corresponding width of the tracker, the elongated portion 312 is retained in the cavity 466 and the insert portion 454 is releasably coupled to the tracker 300.

The insert portion 454 may further comprise a handle 468 on an opposite side of the undercuts 464 that facilitates a user grasping the tracker 300 through the insert portion 454. The user is able to grasp the handle 468 of the insert portion 454 and remove both the insert portion 454 and the tracker 300 from the shell portion 452 simultaneously, and without making direct contact with the tracker 300, aiding sterility. The handle 468 is further arranged such that deflection of the insert portion 454 resulting from pressure the pressure of the user grasping the handle 468 causes the width between opposing undercuts 464 to be decrease. Said differently, the pressure from the user's grasp is partially transferred from the handle 468 to the undercuts 464, which prevents the tracker 300 from being inadvertently decoupled from the insert portion 454. The insert portion 454 also prevents movement of the tracker 300 within the sterile interior 458. Both the insert portion 454 and the tracker 300 are sealed within the sterile interior 458 by the sealing film 456.

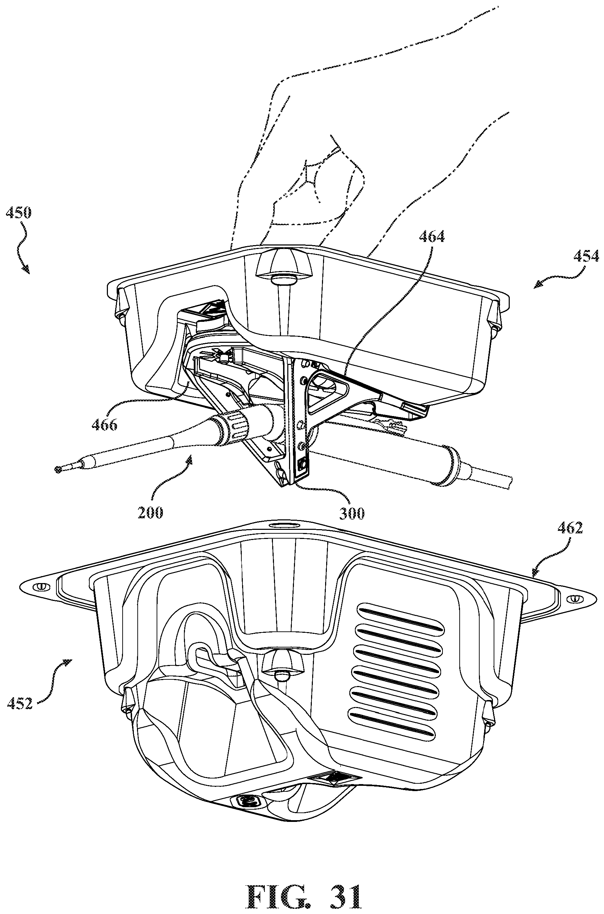

FIGS. 30 and 31 show a user removing the tracker 300 from the sterile interior 458 by grasping the handle 468 to lift both the tracker 300 and the insert portion 454. While still grasping the handle 468 the user is able to manipulate the tracker 300 to assemble the tracker 300 onto the surgical instrument 200. Because the user is not directly handling the tracker 300, the possibility of contaminating the tracker 300 prior to a medical procedure is reduced.

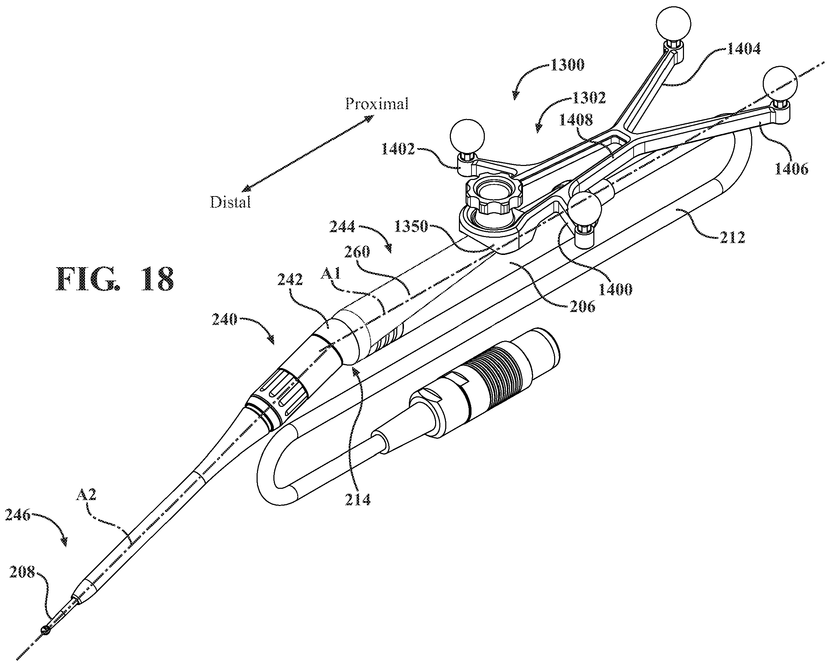

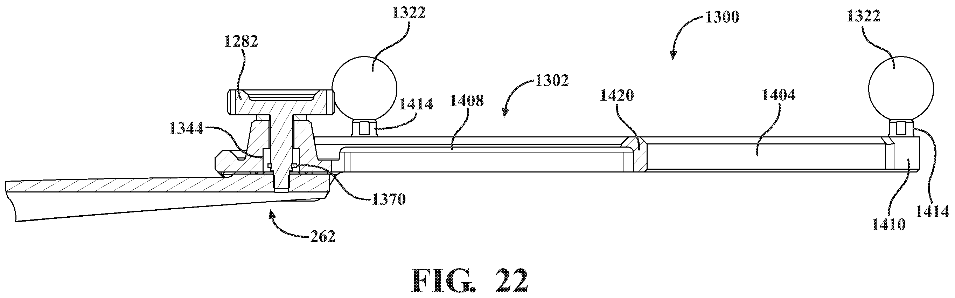

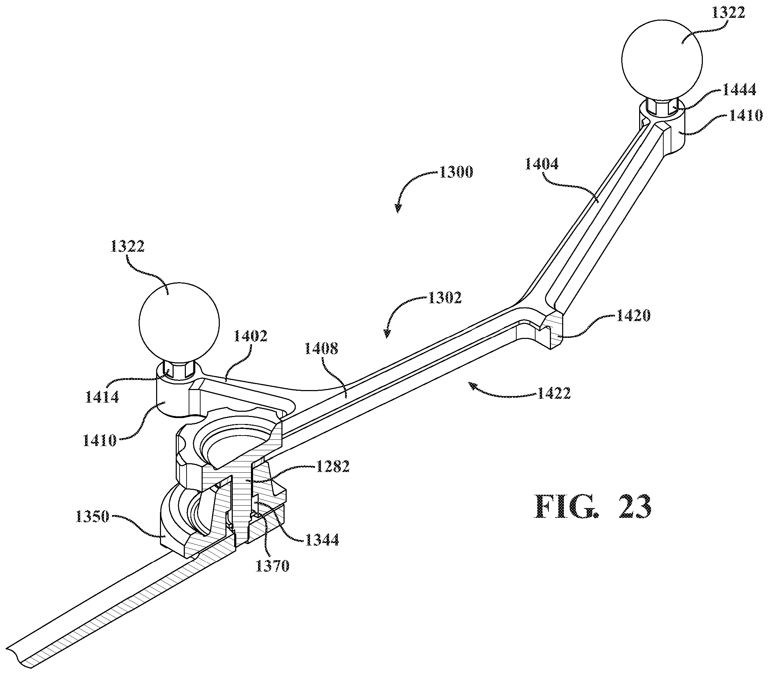

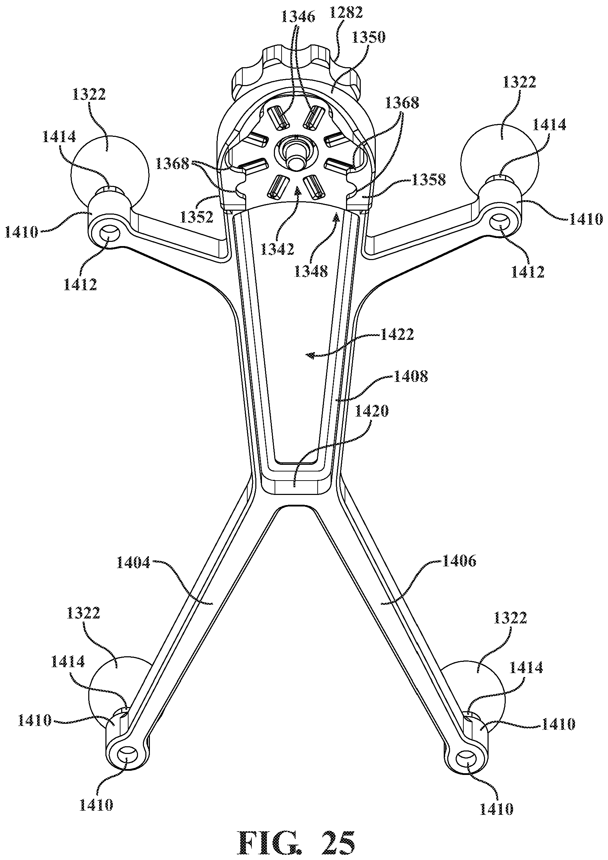

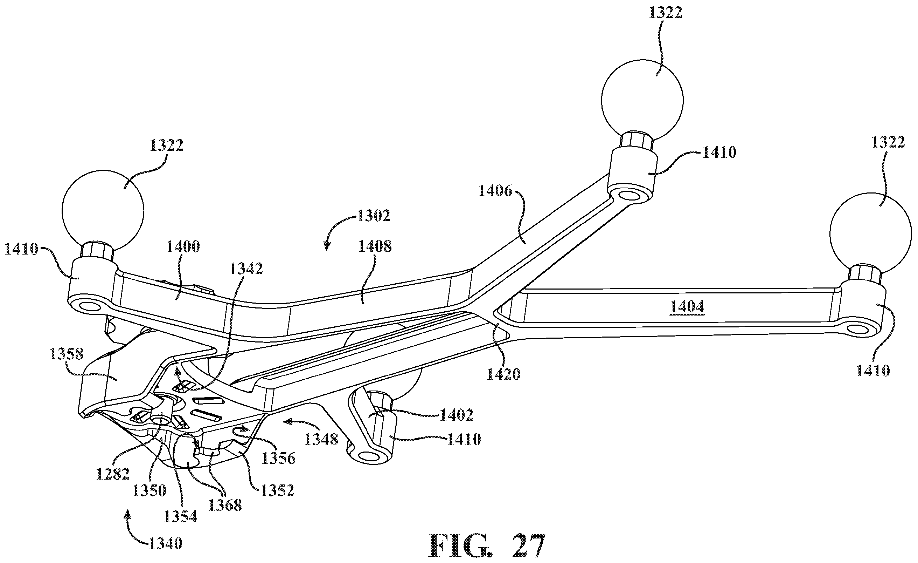

Turning now to FIGS. 18-27, the second exemplary implementation of the navigation tracker 1300 is shown. As will be appreciated from the subsequent description below, the second tracker 1300 is similar to the tracker 300 described above in connection with FIGS. 2-13. As such, the components and structural features of the second implementation of the tracker 1300 that are the same as, or that otherwise correspond to, the first implementation of the tracker 300 are provided with similar reference numerals increased by 1000 (e.g., 300 and 1300). While the differences between these versions will be described in detail, for the purposes of clarity, consistency, and brevity, only certain structural features and components common between these versions will be discussed and depicted in the drawings of the second implementation of the tracker 1300. Here, unless otherwise indicated, the above description of the first implementation of the tracker 300 may be incorporated by reference with respect to the second implementation of the tracker 1300 without limitation.

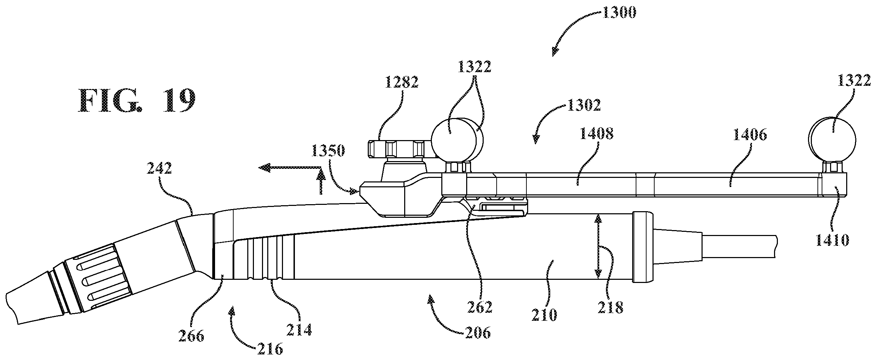

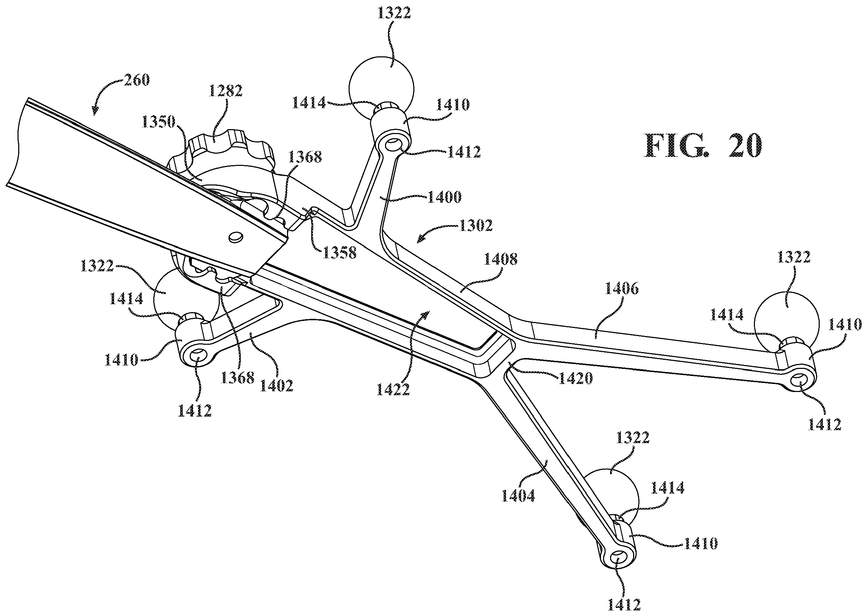

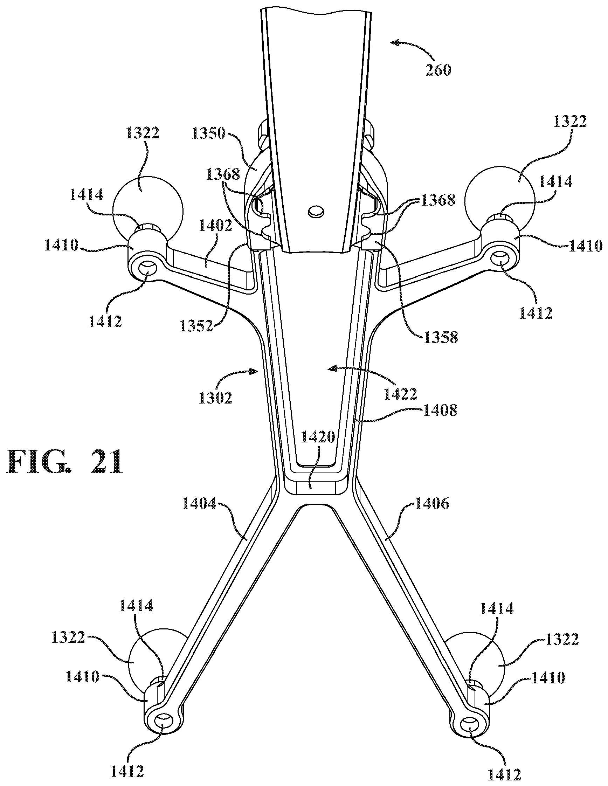

In FIGS. 20 and 21, a close-up perspective view of the surgical instrument 200 and the second implementation of the tracker 1300 is shown. Here, the tracker 1300 may comprise a tracker frame 1302 for supporting a plurality of tracking markers 1322, and an accessory socket 1340 for securing the tracker frame 1302 to the surgical instrument 200. As shown in FIG. 19, the tracker 1300 is shown being removed/assembled to the attachment 240. The tracker 1300 is shown in a partially assembled state with accessory socket 1340 disengaged from the accessory mount 262. The arrows indicate steps of a removal process, in which the tracker 1300 is first lifted to disengage the alignment channels 1346 from the alignment pegs 276 and then moved relative to the accessory mount 262 in a distal direction (i.e., toward the attachment 240). The tracker 1300 is assembled to the attachment 240 in the opposite order by inserting the accessory mount 262 through the socket opening 348 and moving the tracker 1300 in a proximal direction (i.e., away from the attachment 240).

When the navigation system determines the position and orientation of the tracker 1300, it is advantageous that the tracker 1300 remains in the same position relative to the surgical instrument 200 to maximize accuracy. In order to maximize accuracy, it is therefore advantageous for the tracker frame 1302 and the accessory socket 340 to be sufficiently rigid so as to prevent deformation or movement of the markers of the tracker frame 1302 relative to the surgical instrument 200. The desire to minimize bending or deformation of the tracker frame 1302 is limited by the desire to minimize the weight of the tracker frame 1302. Both of the aforementioned desires may also be limited by the desire to increase precision by having a large array of tracking markers 1322, and the desire to eliminate obstruction to the surgeon operating the surgical instrument 200 by having a compact tracker 1300.

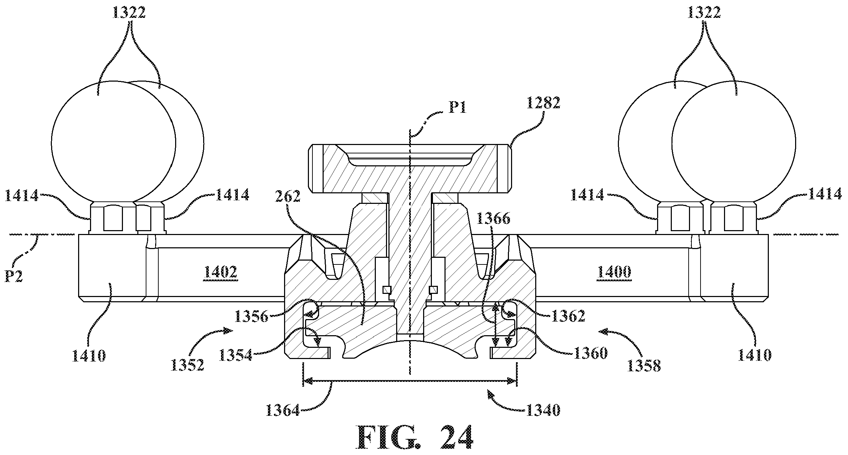

Referring to FIGS. 20-24, in an exemplary configuration, the tracker 1300 may comprise a tracker frame 1302 having at least one arm 1400, 1402, 1404, 1406 protruding therefrom. A tracker plane P2 may be defined parallel to and spaced from the instrument axis A1 of the handheld surgical instrument 200, as shown in FIG. 24. The tracker 1300 may be arranged above the surgical instrument 200 and generally aligned with the tracker plane P2.

In FIG. 21 The at least one arm 1400, 1402, 1404, 1406 may be further defined as a first arm 1400, 1404 and a second arm 1402, 1406, or alternatively a pair of distal arms 1400, 1402, and a pair of proximal arms 1404, 1406. Each arm 1400, 1402, 1404, 1406 protrudes from a body portion 1408 of the tracker frame 1302 to an end spaced from the body portion 1408 and comprises a marker support portion 1410 arranged at the end spaced from the tracker frame 1302. Each marker support portion 1410 may define a post bore 1412, which may be configured to receive a marker post 1414, which may be configured to engage a tracking marker 1322. In the exemplary implementations of the tracker 1300 illustrated the tracking markers 1322 may be reflective spheres or retroreflectors, which reflect light (typically infrared) that is visible to the localizer. It is contemplated that LED tracking markers could be utilized in place of the spherical tracking markers. When configured as LED emitters the marker post 1414 may be omitted and the tracking markers mounted directly on the marker support portions 1410. Other configurations are contemplated.

Each arm 1400, 1402, 1404, 1406 further has a length and a width. A ratio of the length to the width may exceed four (4). Said differently, each of the arms 1400, 1402, 1404, 1406 may have a length that is at least 4x greater than the width. It should be appreciated that each arm 1400, 1402, 1404, 1406 may have a respective length and a respective width, which may differ between each arm, such that the ratio of the length and the width may exceed 4.

One way in which obstruction to the surgeon is minimized is to position the arms 1400, 1402, 1404, 1406 of the tracker 1300 away from a working end of the surgical instrument 200. The arms may be configured with different lengths such that the marker support portions 1410 are arranged with a proximal bias. More specifically, the pair of proximal arms 1404, 1406 extends a first distance from the body portion 1408 and the pair distal arms 1400, 1402 extends a second distance from the body portion 1408, the first distance being greater than the second distance. Said differently, the marker support portions 1410 arranged proximal of the body portion 1408 are spaced further from the body portion 1408 than the marker support portions 1410 arranged distal of the body portion 1408.

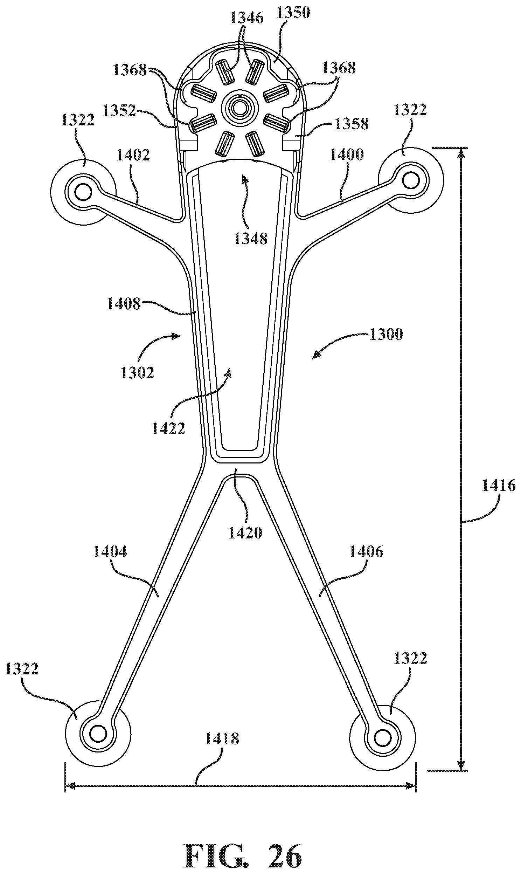

To this end, the tracker 1300 may be configured at different angles relative to the insertion axis A2 such that the marker support portions 1410 are arranged with an axially central bias. Said differently, the marker support portions 1410 are spaced in the proximal to distal direction at a distance greater than the spacing transverse to the insertion axis A2. Best shown in FIG. 26, the tracker 1300 has a length 1416 and a width 1418 defined by the arms 1400, 1402, 1404, 1406, and a ratio of the length 1416 to the width 1418 is greater than 1.5. More specifically the length 1416 that the marker support portions 1410 on the pair of proximal arms 1404, 1406 are spaced from the corresponding marker support portions on the distal arms 1400, 1402 is at least 1.5x greater than the width 1418 that the marker support portions on the first arm 1400, 1404 are spaced from the corresponding marker support portions on the second arm 1402, 1406.

The tracker 1300 may further comprise a bridge portion 1420 spaced in a proximal direction from the body portion 1408 and extending between the first arm 1404 and the second arm 1406. In this way, the bridge portion 1420, the first arm 1404, the second arm 1406, and the tracker frame 1302 cooperate to define an opening 1422 in the tracker 1300. The markers 1322 are generally arranged around and spaced from the opening 1422.

Several instances have been discussed in the foregoing description. However, the aspects discussed herein are not intended to be exhaustive or limit the disclosure to any particular form. Various modifications to these aspects will be readily apparent to those skilled in the art, and the generic principles defined herein may be applied to other aspects without departing from the scope of the disclosure. The terminology that has been used is intended to be in the nature of words of description rather than of limitation. Many modifications and variations are possible in light of the above teachings and the disclosure may be practiced otherwise than as specifically described.

## Claims

1. A tracker attachment (240) for a high-speed drill device (200), the tracker attachment comprising:
an attachment body (242) extending from a proximal end to a distal end, wherein the proximal end is configured for engaging a distal end of a motor body of the high-speed drill device;
a tool chuck (258) supported by the attachment body (242) and configured to receive a material removal tool (208) arranged at a working end of the tracker attachment (240);
a driveshaft (256) supported in the attachment body (242) and operably coupled to the tool chuck (258) to transfer power from the high-speed drill device to the material removal tool (208); **characterised in that** the tracker attachment comprises
a cantilever arm (260) coupled to the attachment body (242) and extending in a proximal direction, wherein a proximal end of the cantilever arm (260) is positioned proximally of the proximal end of the attachment body (242) and is configured to engage with a tracker (300) to removably couple the tracker to the attachment body.

2. The tracker attachment (240) of claim 1, wherein the cantilever arm (260) is coupled to the attachment body (242) using an interference fit.

3. The tracker attachment (240) of claim 1, wherein the cantilever arm (260) is welded to the attachment body (242).

4. The tracker attachment (240) of any one of the preceding claims, wherein the cantilever arm (260) has an arcuate profile.

5. The tracker attachment (240) of any one of the preceding claims, wherein the cantilever arm (260) comprises an accessory mount (262) configured to engage with the tracker (300) to removably couple the tracker to the attachment body (242), the accessory mount (262) comprising:
a trunk portion (272) having a top surface (278) and defining a clamping hole (280) in the top surface (272); and
a first lateral wing portion (274A) and a second lateral wing portion (274B) protruding from opposing sides of the trunk portion (272) transverse to the top surface (278), wherein the first lateral wing portion (274A) has a first lateral face (284A), and the second lateral wing portion (274B) has a second lateral face (284B), wherein the first lateral face (284A) and the second lateral face (284B) are symmetrically angled about a longitudinal plane of the high-speed drill device.

6. The tracker attachment of claim 5, wherein the accessory mount (262) further comprises one or more alignment pegs (276) protruding from the top surface (278).

7. The tracker attachment (240) of any one of claims 5 or 6, wherein the cantilever arm (260) and the accessory mount (262) are integrally formed.

8. The tracker attachment (240) of any one of the preceding claims, wherein
the attachment body (242) further comprises a coupling portion arranged at the proximal end of the attachment body (242), wherein the coupling portion is engageable with the motor body of the high-speed drill device and a mount flange (252).

9. A system for tracking a position of a rotary cutting tool, the system comprising:
a rotary cutting tool (208);
a surgical handpiece (206) including a motor body;
the tracker attachment (240) of any one of claims 1 to 8; and
a tracker (300) comprising:
a tracker frame (302) configured to engage the accessory mount (262); and
at least three optical markers coupled to the tracker frame (302); and
wherein the tracker (300) is arranged proximally of the attachment body (242) when the tracker frame (302) is engaged with the accessory mount (262).

10. The system of claim 9, wherein the tracker (300) is spaced from the surgical handpiece (206) when the tracker frame (302) is engaged with the accessory mount (262).

11. The system of any one of claims 9 or 10, wherein the tracker frame (302) radially surrounds the surgical handpiece (206) when the tracker frame (302) is engaged with the accessory mount (262).

12. The system of any one of claims 9 to 11, wherein the tracker frame (302) defines an instrument aperture, and wherein the surgical handpiece (206) is disposed in the instrument aperture when the tracker frame (302) is engaged with the accessory mount (262).

13. The system of any one of claims 9 to 12, wherein the accessory mount (262) is spaced proximally of the proximal end of the attachment body (242) at a first distance and spaced radially from the surgical handpiece (206) at a second distance, and wherein the first distance is greater than the second distance.

14. The system of claim 13, wherein the first distance is at least 1.5 times greater than the second distance.

15. The system of claim 13, wherein the first distance is at least 2 times greater than the second distance.

## Patentansprüche

1. Tracker-Aufsatz (240) für eine Hochgeschwindigkeitsbohrvorrichtung (200), wobei der Tracker-Aufsatz umfasst:
einen Aufsatzkörper (242), der sich von einem proximalen Ende zu einem distalen Ende erstreckt, wobei das proximale Ende zum Eingriff mit einem distalen Ende eines Motorkörpers der Hochgeschwindigkeitsbohrvorrichtung eingerichtet ist;
ein Werkzeugfutter (258), das vom Aufsatzkörper (242) getragen wird und dazu eingerichtet ist, ein an einem Arbeitsende des Tracker-Aufsatzes (240) angeordnetes Materialabtragswerkzeug (208) aufzunehmen;
eine Antriebswelle (256), die im Aufsatzkörper (242) gelagert und betriebsmäßig mit dem Werkzeugfutter (258) gekoppelt ist, um Kraft von der Hochgeschwindigkeitsbohrvorrichtung auf das Materialabtragswerkzeug (208) zu übertragen;
**dadurch gekennzeichnet, dass** der Tracker-Aufsatz einen Kragarm (260) umfasst, der mit dem Aufsatzkörper (242) gekoppelt ist und sich in eine proximale Richtung erstreckt, wobei ein proximales Ende des Kragarms (260) proximal zum proximalen Ende des Aufsatzkörpers (242) positioniert ist und dazu eingerichtet ist, mit einem Tracker (300) in Eingriff zu kommen, um den Tracker lösbar mit dem Aufsatzkörper zu koppeln.

2. Tracker-Aufsatz (240) nach Anspruch 1, wobei der Kragarm (260) mittels einer Presspassung mit dem Aufsatzkörper (242) gekoppelt ist.

3. Tracker-Aufsatz (240) nach Anspruch 1, wobei der Kragarm (260) an den Aufsatzkörper (242) geschweißt ist.

4. Tracker-Aufsatz (240) nach einem der vorhergehenden Ansprüche, wobei der Kragarm (260) ein bogenförmiges Profil aufweist.

5. Tracker-Aufsatz (240) nach einem der vorhergehenden Ansprüche, wobei der Kragarm (260) eine Zubehörhalterung (262) umfasst, die dazu eingerichtet ist, mit dem Tracker (300) in Eingriff zu kommen, um den Tracker lösbar mit dem Aufsatzkörper (242) zu koppeln, wobei die Zubehörhalterung (262) umfasst:
einen Rumpfabschnitt (272), der eine obere Fläche (278) aufweist und ein Klemmloch (280) in der oberen Fläche (278) definiert; und
einen ersten seitlichen Flügelabschnitt (274A) und einen zweiten seitlichen Flügelabschnitt (274B), die von gegenüberliegenden Seiten des Rumpfabschnitts (272) quer zur oberen Fläche (278) vorstehen, wobei der erste seitliche Flügelabschnitt (274A) eine erste seitliche Fläche (284A) aufweist und der zweite seitliche Flügelabschnitt (274B) eine zweite seitliche Fläche (284B) aufweist, wobei die erste seitliche Fläche (284A) und die zweite seitliche Fläche (284B) symmetrisch um eine Längsebene der Hochgeschwindigkeitsbohrvorrichtung gewinkelt sind.

6. Tracker-Aufsatz nach Anspruch 5, wobei die Zubehörhalterung (262) ferner einen oder mehrere Ausrichtungszapfen (276) umfasst, die von der oberen Fläche (278) vorstehen.

7. Tracker-Aufsatz (240) nach einem der Ansprüche 5 oder 6, wobei der Kragarm (260) und die Zubehörhalterung (262) einstückig ausgebildet sind.

8. Tracker-Aufsatz (240) nach einem der vorhergehenden Ansprüche, wobei der Aufsatzkörper (242) ferner einen Kopplungsabschnitt umfasst, der am proximalen Ende des Aufsatzkörpers (242) angeordnet ist, wobei der Kopplungsabschnitt mit dem Motorkörper der Hochgeschwindigkeitsbohrvorrichtung und einem Montageflansch (252) in Eingriff bringbar ist.

9. System zum Verfolgen einer Position eines rotierenden Schneidwerkzeugs, wobei das System umfasst:
ein rotierendes Schneidwerkzeug (208);
ein chirurgisches Handstück (206), das einen Motorkörper enthält;
den Tracker-Aufsatz (240) nach einem der Ansprüche 1 bis 8; und
einen Tracker (300), umfassend:
einen Tracker-Rahmen (302), der dazu eingerichtet ist, mit der Zubehörhalterung (262) in Eingriff zu kommen; und
mindestens drei optische Marker, die mit dem Tracker-Rahmen (302) gekoppelt sind; und
wobei der Tracker (300) proximal zum Aufsatzkörper (242) angeordnet ist, wenn der Tracker-Rahmen (302) mit der Zubehörhalterung (262) in Eingriff steht.

10. System nach Anspruch 9, wobei der Tracker (300) vom chirurgischen Handstück (206) beabstandet ist, wenn der Tracker-Rahmen (302) mit der Zubehörhalterung (262) in Eingriff steht.

11. System nach einem der Ansprüche 9 oder 10, wobei der Tracker-Rahmen (302) das chirurgische Handstück (206) radial umgibt, wenn der Tracker-Rahmen (302) mit der Zubehörhalterung (262) in Eingriff steht.

12. System nach einem der Ansprüche 9 bis 11, wobei der Tracker-Rahmen (302) eine Instrumentenöffnung definiert und wobei das chirurgische Handstück (206) in der Instrumentenöffnung angeordnet ist, wenn der Tracker-Rahmen (302) mit der Zubehörhalterung (262) in Eingriff steht.

13. System nach einem der Ansprüche 9 bis 12, wobei die Zubehörhalterung (262) proximal zum proximalen Ende des Aufsatzkörpers (242) in einem ersten Abstand beabstandet ist und radial vom chirurgischen Handstück (206) in einem zweiten Abstand beabstandet ist, und wobei der erste Abstand größer ist als der zweite Abstand.

14. System nach Anspruch 13, wobei der erste Abstand mindestens 1,5-mal größer ist als der zweite Abstand.

15. System nach Anspruch 13, wobei der erste Abstand mindestens 2-mal größer ist als der zweite Abstand.

## Revendications

1. Accessoire de suivi (240) pour un dispositif de perçage à grande vitesse (200), l'accessoire de suivi comprenant:
un corps de fixation (242) s'étendant d'une extrémité proximale à une extrémité distale, dans lequel l'extrémité proximale est conçue pour entrer en prise avec une extrémité distale d'un corps de moteur du dispositif de perçage à grande vitesse;
un mandrin d'outil (258) supporté par le corps de fixation (242) et conçu pour recevoir un outil d'enlèvement de matière (208) disposé à une extrémité de travail de l'accessoire de suivi (240);
un arbre de transmission (256) supporté dans le corps de fixation (242) et couplé fonctionnellement au mandrin d'outil (258) pour transférer la puissance du dispositif de perçage à grande vitesse à l'outil d'enlèvement de matière (208); **caractérisé en ce que** l'accessoire de suivi comprend
un bras en porte-à-faux (260) couplé au corps de fixation (242) et s'étendant dans une direction proximale, dans lequel une extrémité proximale du bras en porte-à-faux (260) est positionnée de manière proximale par rapport à l'extrémité proximale du corps de fixation (242) et est conçue pour venir s'insérer dans un dispositif de suivi (300) afin d'accoupler de manière amovible avec le dispositif de suivi au corps de fixation.

2. Accessoire de suivi (240) selon la revendication 1, dans lequel le bras en porte-à-faux (260) est couplé au corps de fixation (242) à l'aide d'un ajustement serré.

3. Accessoire de suivi (240) selon la revendication 1, dans lequel le bras en porte-à-faux (260) est soudé au corps de fixation (242).

4. Accessoire de suivi (240) selon l'une quelconque des revendications précédentes, dans lequel le bras en porte-à-faux (260) présente un profil arqué.

5. Accessoire de suivi (240) selon l'une quelconque des revendications précédentes, dans lequel le bras en porte-à-faux (260) comprend un support d'accessoire (262) conçu pour s'assembler avec le dispositif de suivi (300) afin de coupler de manière amovible le dispositif de suivi au corps de fixation (242), le support d'accessoire (262) comprenant:
une partie tronc (272) ayant une surface supérieure (278) et formant un trou de serrage (280) dans la surface supérieure (272); et
une première partie aile latérale (274A) et une deuxième partie aile latérale (274B) faisant saillie des côtés opposés de la partie tronc (272) transversalement à la surface supérieure (278), la première partie aile latérale (274A) comportant une première face latérale (284A), et la deuxième partie aile latérale (274B) présentant une deuxième face latérale (284B), la première face latérale (284A) et la deuxième face latérale (284B) étant inclinées symétriquement autour d'un plan longitudinal du dispositif de perçage à grande vitesse.

6. Accessoire de suivi (240) selon la revendication 5, dans lequel le support d'accessoire (262) comprend en outre une ou plusieurs chevilles d'alignement (276) faisant saillie de la surface supérieure (278).

7. Accessoire de suivi (240) selon l'une quelconque des revendications 5 ou 6, dans lequel le bras en porte-à-faux (260) et le support d'accessoire (262) sont formés d'un seul tenant.

8. Accessoire de suivi (240) selon l'une quelconque des revendications précédentes, dans lequel le corps de fixation (242) comprend en outre une partie d'accouplement disposée au niveau de
l'extrémité proximale du corps de fixation (242), dans lequel la partie d'accouplement
peut entrer en prise avec le corps de moteur du dispositif de perçage à grande vitesse et un rebord
de montage (252).

9. Système de suivi d'une position d'un outil de coupe rotatif, le système comprenant:
un outil de coupe rotatif (208);
une pièce à main chirurgicale (206) comprenant un corps de moteur;
l'accessoire de suivi (240) selon l'une quelconque des revendications 1 à 8; et
un dispositif de suivi (300) comprenant:
un cadre de dispositif de suivi (302) conçu pour venir en prise avec le support d'accessoire (262); et
au moins trois marqueurs optiques couplés au cadre de dispositif de suivi (302); et
dans lequel le dispositif de suivi (300) est disposé à proximité du corps de fixation (242) lorsque le cadre de dispositif de suivi (302) est en prise avec le support accessoire (262).

10. Système selon la revendication 9, dans lequel le dispositif de suivi (300) est espacé de la pièce à main chirurgicale (206) lorsque le cadre de dispositif de suivi (302) est en prise avec le support d'accessoire (262).

11. Système selon l'une quelconque des revendications 9 ou 10, dans lequel le cadre de suivi (302) entoure radialement la pièce à main chirurgicale (206) lorsque le cadre de dispositif de suivi (302) est en prise avec le support d'accessoire (262).

12. Système selon l'une quelconque des revendications 9 à 11, dans lequel le cadre de suivi (302) définit une ouverture pour instrument, et dans lequel la pièce à main chirurgicale (206) est disposée dans l'ouverture pour instrument lorsque le cadre de dispositif de suivi (302) est en prise avec le support d'accessoire (262).

13. Système selon l'une quelconque des revendications 9 à 12, dans lequel le support d'accessoire (262) est espacé de manière proximale de l'extrémité proximale du corps de fixation (242) à une première distance et espacé radialement de la pièce à main chirurgicale (206) à une deuxième distance, et dans lequel la première distance est supérieure à la deuxième distance.

14. Système selon la revendication 13, dans lequel la première distance est au moins 1,5 fois supérieure à la deuxième distance.

15. Système selon la revendication 13, dans lequel la première distance est au moins 2 fois supérieure à la deuxième distance.
